# EUROPÄISCHE PATENTSCHRIFT

(11) **EP 2 531 118 B1**
(45) Veröffentlichungstag und Bekanntmachung des Hinweises auf die Patenterteilung: **23.04.2014**
(21) Anmeldenummer: 11702796.1
(22) Anmeldetag: 04.02.2011
(51) Int. Cl.: A61B 17/221, A61B 17/3207

(54) **MEDIZINISCHE VORRICHTUNG ZUM LÖSEN VON KONKREMENTEN, VERFAHREN ZUM HERSTELLEN EINER DERARTIGEN VORRICHTUNG, BEHANDLUNGSSYSTEM MIT EINER DERARTIGEN VORRICHTUNG UND VERFAHREN ZUM HERSTELLEN EINES BEHANDLUNGSSYSTEMS**
MEDICAL DEVICE FOR LOOSENING CONCRETIONS, METHOD FOR PRODUCING SUCH A DEVICE, TREATMENT SYSTEM COMPRISING SUCH A DEVICE AND METHOD FOR PRODUCING A TREATMENT SYSTEM
DISPOSITIF MÉDICAL POUR DÉGAGER DES CALCULS, PROCÉDÉ DE FABRICATION D'UN TEL DISPOSITIF, SYSTÈME DE TRAITEMENT AVEC UN TEL DISPOSITIF ET PROCÉDÉ DE FABRICATION D'UN SYSTÈME DE TRAITEMENT

(30) Priorität: 30.06.2010 DE 102010025661; 05.02.2010 DE 102010006962
(43) Veröffentlichungstag der Anmeldung: 12.12.2012
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2011/000529
(87) Internationale Veröffentlichungsnummer: WO 2011/095352

(56) Entgegenhaltungen:
- WO-A2-2007/089897
- US-A- 6 077 274
- US-A1- 2002 010 487
- US-A1- 2003 208 215
- US-A1- 2007 208 361

## Beschreibung

Die Erfindung betrifft eine medizinische Vorrichtung zum Lösen von Konkrementen, ein Verfahren zum Herstellen einer derartigen Vorrichtung, ein Behandlungssystem mit einer derartigen Vorrichtung und ein Verfahren zum Herstellen eines Behandlungssystems.

Aus der Praxis sind allgemein medizinische Vorrichtungen bekannt, die zum Entfernen bzw. Lösen von Blutgerinnseln oder Plaque-Bestandteilen aus Blutgefäßen dienen. Derartige Vorrichtungen bzw. Instrumente umfassen einen Führungsdraht, der innerhalb eines Zufuhrkatheters geführt ist. Am distalen Ende bzw. an der Spitze des Führungsdrahtes ist eine korbartige Gitterstruktur angeordnet. Die korbartige Gitterstruktur kann beispielsweise ein Drahtgeflecht umfassen. Der aufgespannte Korb dient im Blutgefäß einerseits als Filter, der ein Abfließen von Thrombuspartikeln oder Plaque-Partikeln verhindert. Andererseits ermöglicht die korbartig aufgespannte Gitterstruktur ein Einfangen von Blutgerinnseln oder sonstigen Fremdkörpern.

Im Allgemeinen ist die Gitterstruktur radial expandierbar, so dass sich die Gitterstruktur beim Verlassen des Katheters in einem Blutgefäß aufspannt. Aufgrund der gitterartigen bzw. netzartigen Anordnung einzelner Elemente der Gitterstruktur bewirkt die radiale Expansion des distal am Führungsdraht angeordneten Korbes gleichzeitig eine Verkürzung der Gitterstruktur in Längsrichtung.

Blutgerinnsel, insbesondere Thromben oder Plaque können sich fest an die Gefäßwand eines Blutgefäßes anlagern. Die Ablösung derartiger Ablagerungen ist in der Praxis aufwändig. Neben der medikamentösen Ablösung der Ablagerungen ist es bekannt, die korbartige Gitterstruktur als mechanisches Lösemittel zu nutzen. Insbesondere kann die Gitterstruktur zum Abschaben von Ablagerungen genutzt werden. Dazu wird der Führungsdraht am proximalen Ende vom Anwender rotiert, so dass sich am distalen Ende des Führungsdrahtes, insbesondere an der korbartigen Gitterstruktur, eine Rotation einstellt, die ein mechanisches Ablösen der Ablagerung bewirkt.

Die mechanische Ablösung von Ablagerungen ist schwierig, da der relativ dünne Führungsdraht durch manuelle Betätigung schwer rotierbar ist. Insbesondere unter sterilen Bedingungen ist es erforderlich, dass der Anwender Handschuhe trägt, die die Übertragung eines Drehmoments auf den Führungsdraht zusätzlich erschweren. Ferner bewirkt die Rotation des Führungsdrahts am proximalen Ende aufgrund der Länge des Führungsdrahtes und seiner Elastizität zunächst eine Torsion des Führungsdrahtes, die erst zeitlich versetzt zu einer Rotation der korbartigen Gitterstruktur führt. Dabei besteht die Gefahr, dass die Torsion des Führungsdrahtes ruckartig in eine Rotationsbewegung der Gitterstruktur umgesetzt wird. Überdies ist der Grad der Rotation der distal angeordneten Gitterstruktur aufgrund der Elastizität des Führungsdrahtes schwer kontrollierbar. Der am proximalen Ende des Führungsdrahts tätige Anwender erhält eine unzureichende Rückmeldung über den momentanen Rotationszustand der distal angeordneten Gitterstruktur. Durch die Elastizität und die Torsionsneigung des Führungsdrahtes besteht außerdem die Gefahr, daß die Rotation, die vom Anwender am proximalen Ende des Führungsdrahts aufgebracht wird, derart in eine Torsion des Führungsdrahts umgesetzt wird, dass der Führungsdraht verkürzt wird. Das kann zu einer längsaxialen Lageänderung der distal angeordneten Gitterstruktur führen, wobei die Lageänderung, insbesondere die zeitliche Dynamik der Lageänderung, durch den Anwender kaum kontrollierbar ist.

Dokumente US 2002/010487 A, US 6 077 274 A, WO 2007/089897 A und US 2007/208361 A offenbaren medizinische Vorrichtungen zum Lösen von Konkrementen in Körperhohlorganen mit wenigstens einer rotationssymmetrischen Gitterstruktur mit sich kreuzenden Filamenten oder sich kreuzenden Gitterstegen. Diese Vorrichtungen werden entweder am proximalen Ende oder mittels Einkerbungen an einem Umhüllungsschlauch, in Rotation versetzt.

Die Aufgabe der Erfindung besteht darin, eine medizinische Vorrichtung anzugeben, die ein gezieltes Ablösen von Konkrementen ermöglicht und eine verbesserte Handhabbarkeit aufweist. Ferner besteht die Aufgabe der Erfindung darin, ein Verfahren zum Herstellen einer derartigen Vorrichtung, ein Behandlungssystem mit einer derartigen Vorrichtung und ein Verfahren zum Herstellen eines Behandlungssystems anzugeben.

Erfindungsgemäß wird diese Aufgabe im Hinblick auf die medizinische Vorrichtung durch den Gegenstand des Patentanspruchs 1 alternativ durch den Gegenstand des Anspruchs 17, im Hinblick auf das Herstellverfahren für eine medizinische Vorrichtung nach Anspruch 1 durch den Gegenstand des Patentanspruchs 16, im Hinblick auf das Behandlungssystem durch den Gegenstand des Patentanspruchs 18 und im Hinblick auf das Herstellverfahren für ein Behandlungssystem durch den Gegenstand des Patentanspruchs 19 gelöst.

Die Erfindung beruht auf dem Gedanken, eine medizinische Vorrichtung zum Lösen von Konkrementen in Körperhohlorganen mit wenigstens einer rotationssymmetrischen Gitterstruktur anzugeben, die mit einem Halteelement drehfest verbunden ist. Die Gitterstruktur weist wenigstens zwei um eine gemeinsame Rotationsachse gewundene und sich kreuzende Filamente auf. Die Filamente sind an wenigstens zwei in Längsrichtung der Gitterstruktur beabstandet angeordneten Verbindungsstellen miteinander verbunden. Dabei bildet ein erstes Filament einen ersten Flechtwinkel α und ein zweites Filament einen zweiten Flechtwinkel β. Der zweite Flechtwinkel β ist zumindest abschnittsweise derart von dem ersten Flechtwinkel α verschieden, dass eine erste Verbindungsstelle beim Übergang der Gitterstruktur von einem radial komprimierten Zustand in einen radial expandierten Zustand, oder umgekehrt, gegenüber einer zweiten Verbindungsstelle um die gemeinsame Rotationsachse verdreht wird.

Der Erfindung liegt insbesondere die Idee zugrunde, in der rotationssymmetrischen Gitterstruktur eine Rotation auszulösen, wenn die Gitterstruktur vom komprimierten in den expandierten Zustand überführt wird. Die Verdrehung der ersten Verbindungsstelle gegenüber der zweiten Verbindungsstelle erfolgt während des Übergangs der Gitterstruktur vom radial komprimierten in den radial expandierten Zustand. Umgekehrt erfolgt die Rotation bzw. Verdrehung auch beim Übergang vom expandierten in den komprimierten Zustand. Im zeitlichen Verlauf der Zustandsänderung der Gitterstruktur wird eine dynamische Rotation der Verbindungsstellen zueinander bewirkt.

Die Rotation wird im Wesentlichen selbsttätig erreicht. Das bedeutet in der Praxis, dass eine längsaxiale Lageänderung der Vorrichtung, beispielsweise relativ zu einem Zufuhrsystem, insbesondere einem Katheter, ausreicht, um die Rotation bzw. die abschnittsweise Rotation der Gitterstruktur zu bewirken. Die längsaxiale Bewegung des Halteelements ist durch einen Anwender relativ einfach und leicht kontrollierbar. Insbesondere wird eine längsaxiale Bewegung des Halteelements, die am proximalen Ende des Halteelements aufgebracht wird, vergleichsweise direkt in eine längsaxiale Bewegung des distalen Endes des Halteelements der Gitterstruktur übertragen. Die Kontrollierbarkeit bzw. Handhabbarkeit der medizinischen Vorrichtung wird somit insgesamt gesteigert.

Überdies bewirken die unterschiedlichen Flechtwinkel α, β der sich kreuzenden Filamente eine definierte, also festgelegte, Relativrotation der beiden Verbindungsstellen in Abhängigkeit von der längsaxialen Längenänderung der Gitterstruktur. Das bedeutet konkret, dass der Rotationsgrad der Gitterstruktur bzw. der Verbindungsstellen zueinander durch die unterschiedlichen Flechtwinkel vom Abstand der Verbindungsstellen abhängig sind. Durch die Wahl der Flechtwinkel α, β kann somit der Rotationsgrad relativ zur Abstandsänderung zwischen den Verbindungsstellen eingestellt werden.

Ein weiterer Vorteil der längsaxialen bzw. translatorischen Längenänderung der Gitterstruktur besteht darin, dass die Längenänderung relativ einfach zu kontrollieren ist und zwar durch den Aufweitungsgrad bzw. den Komprimierungsgrad der durch die Filamente gebildeten Spiralen. Damit lässt sich die Relativrotation bzw. die Verdrehung der Gitterstruktur gut steuern. Die Verdrehung wird durch eine relative Längsbewegung der Gitterstruktur, insbesondere des Geflechts, bezüglich des Katheters erreicht. Beispielsweise kann die Gitterstruktur ortsfest gehalten und ein Zufuhrsystem, insbesondere ein Katheter, gezogen oder das Zufuhrsystem bzw. der Katheter ortsfest gehalten und die Gitterstruktur geschoben werden, um die Relativbewegung zwischen Zufuhrsystem/Katheter und Gitterstruktur zu erreichen.

Die Filamente sind an den Verbindungsstellen nicht zwangsläufig form- oder kraftschlüssig miteinander verbunden. Ausdrücklich können die Filamente an den Verbindungsstellen auch stoffschlüssig, insbesondere einstückig verbunden sein. Das bedeutet, dass zumindest eine Verbindungsstelle beispielsweise als Umlenkstelle eines Drahtelements ausgebildet sein kann, wobei das Drahtelement in einer Axialrichtung der Gitterstruktur ein das erste Filament und in gegenläufiger Axialrichtung der Gitterstruktur das zweite Filament bildet. Insgesamt können beide Filamente durch ein einziges Drahtelement gebildet sein, das an einem beliebigen Punkt der Gitterstruktur, der im Rahmen der Anmeldung als Verbindungsstelle bezeichnet wird, in axialer Richtung der Gitterstruktur umgelenkt bzw. umgebogen und entlang der Gitterstruktur zurückgeführt wird. Die beiden durch den Umlenkpunkt bzw. die Verbindungsstelle getrennten Abschnitte des Drahtelements bilden jeweils das erste Filament und das zweite Filament, wobei die Filamente in unterschiedlichen Axialrichtungen um die gemeinsame Rotationsachse gewunden sind derart, dass sich das erste und das zweite Filament kreuzen. Vorzugsweise sind die Filamente gegenläufig bzw. gegensinnig, also mit unterschiedlichen Uhrzeigerrichtungen, um die gemeinsame Rotationsachse gewunden.

Filamente können Drähte, Kunststoffelemente oder allgemein längliche Elemente sein, die die Gitterstruktur bilden. Die Elemente können lasergeschnittene oder miteinander verflochtene Elemente bzw. Filamente sein. Die Materialwahl ist nicht eingeschränkt. Bevorzugt sind Materialien für selbstexpandierende Gitterstrukturen, d.h. Formgedächtnismaterialien, beispielsweise Nickel-Titanlegierungen, insbesondere Nitinol. Möglich sind auch Polymerfasern, insbesondere biodegradierbare Polymere, aus denen die Elemente hergestellt sind. Biologisch abbaubare Materialien können auch metallische Materialien sein, wie Magnesium oder auf Magnesium basierende Materialien.

Die Konfiguration der Flechtwinkel α, β bezieht sich im Rahmen der Anmeldung auf den Ruhezustand der Vorrichtung. Die Bestimmung des Flechtwinkels wird bei der erfindungsgemäßen Vorrichtung im entspannten Zustand, also ohne Einwirkung äußerer Kräfte, vorgenommen. Dieser Zustand kann beispielsweise dem Herstellzustand entsprechen. Zur Bestimmung der Flechtwinkel α, β ist die Vorrichtung ferner in axialer Richtung geradlinig ausgerichtet. Die erfindungsgemäße Konfiguration der Gitterstruktur mit unterschiedlichen Flechtwinkeln wird im Unterschied zu bekannten Gitterstrukturen, die sich kreuzende Gitterelemente mit jeweils dem gleichen Flechtwinkel aufweisen, als asymmetrisch bezeichnet. Dabei wird im Rahmen der Anmeldung als Flechtwinkel der Winkel bezeichnet, der zwischen einem Filament und einer senkrechten Projektion der Rotationsachse auf die Umfangsebene der Gitterstruktur gebildet ist. Der Vergleich der Flechtwinkel α, β erfolgt auf derselben axialen Höhe der Gitterstruktur, d.h. in einer senkrecht zur Rotationsachse orientierten Querschnittsebene der Gitterstruktur.

Bei einer Durchmesser- oder Längenänderung verhalten sich separate Filamente, die unterschiedliche Flechtwinkel aufweisen, verschieden. Durch die asymmetrische Gitterstruktur bzw. die unterschiedlichen Flechtwinkel der Filamente wird erreicht, dass sich die Filamente zwischen den beiden Verbindungsstellen bei der Expansion oder Komprimierung der Gitterstruktur um die gemeinsame Rotationsachse drehen. Konkret erfährt eine der Verbindungsstellen gegenüber der anderen Verbindungsstelle eine Änderung der Winkelposition bezüglich der gemeinsamen Rotationsachse. Die Gitterstruktur zwischen den beiden Verbindungsstellen tordiert um die gemeinsame Rotationsachse. Zwischen den beiden Verbindungsstellen erfolgt eine Torsion der Gitterstruktur um die gemeinsame Rotationsachse. Die Torsion wird im Wesentlichen durch innere Kräfte der Gitterstruktur bewirkt, die auf die unterschiedlichen Flechtwinkeln zurückzuführen sind.

Die Rotation der Verbindungsstellen zueinander bzw. die Torsion der Gitterstruktur zwischen den Verbindungsstellen kann vorteilhafterweise durch eine längsaxiale Bewegung der Gitterstruktur, beispielsweise durch sukzessive Freigabe der Gitterstruktur aus einem Katheter, bewirkt werden. Insbesondere kann die Rotation der Verbindungsstellen zueinander durch eine Zug- und/oder Schubbewegung des Halteelements durch den Anwender ausgelöst werden. Das Halteelement weist daher vorzugsweise ein flexibles Führungsmittel, insbesondere einen Führungsdraht, auf. Die Flexibilität des Führungsmittels ermöglicht die Zufuhr der medizinischen Vorrichtung in gekrümmte Körperhohlräume, beispielsweise Blutgefäße.

Bei einer bevorzugten Ausführungsform der erfindungsgemäßen medizinischen Vorrichtung weist die Gitterstruktur ein erstes axiales und ein zweites axiales Ende auf, wobei das erste axiale Ende mit dem Halteelement drehfest verbunden und das zweite axiale Ende frei angeordnet ist. Es ist nicht ausgeschlossen, dass das Halteelement drehfest mit dem zweiten axialen Ende der Gitterstruktur verbunden und das erste axiale Ende frei angeordnet ist. Dabei kann das erste axiale Ende in Relation zum Standpunkt des Anwenders proximal und das zweite axiale Ende distal angeordnet sein. Die Verbindung des Haltemittels mit dem ersten axialen Ende ist im Hinblick auf die Handhabbarkeit, insbesondere den Komprimierungsgrad bzw. die Crimpbarkeit der Gitterstruktur, vorteilhaft. Durch die Verbindung des Halteelements mit dem ersten axialen Ende, insbesondere dem proximalen Ende, der Gitterstruktur, wird vermieden, dass das Halteelement durch die Gitterstruktur hindurchgeführt ist, so dass sich die Gitterstruktur insgesamt auf einen kleineren komprimierten Querschnittsdurchmesser crimpen bzw. reduzieren lässt.

Die erste Verbindungsstelle kann vorteilhafterweise am zweiten axialen Ende, und die zweite Verbindungsstelle am ersten axialen Ende der Gitterstruktur angeordnet sein. Auf diese Weise wird erreicht, dass die gesamte Gitterstruktur beim Übergang vom komprimierten in den expandierten Zustand oder umgekehrt, um die gemeinsame Rotationsachse tordiert. Somit kann die gesamte Länge der Gitterstruktur vorteilhaft zum Lösen von Konkrementen eingesetzt werden. Es ist alternativ auch möglich, dass zumindest eine der Verbindungsstellen zwischen den axialen Enden der Gitterstruktur, insbesondere an beliebiger Stelle, angeordnet ist. Beispielsweise kann die zweite Verbindungsstelle am ersten axialen Ende und die erste Verbindungsstelle beabstandet vom zweiten axialen Ende angeordnet sein. Ferner können auch beide Verbindungsstellen beabstandet von den axialen Enden der Gitterstruktur angeordnet sein. Grundsätzlich sind alle Alternativen möglich, bei denen die Verbindungsstellen zueinander beabstandet angeordnet sind.

Die Rotation der Verbindungsstellen zueinander bzw. die Torsion des zwischen den Verbindungsstellen angeordneten Gitterstrukturabschnitts wird besonders einfach und effizient erreicht, wenn das erste Filament mit dem zweiten Filament verflochten ist. Dadurch wird eine geflochtene Gitterstruktur gebildet. Zwar ist nicht ausgeschlossen, dass die Gitterstruktur durch zwei sich vollständig überlagernde, gegenläufig gewundene Filamentspiralen oder durch eine lasergeschnittene Struktur gebildet ist. Die Rotations- bzw. Torsionsfunktion ist jedoch mit einer geflochtenen Gitterstruktur besonders effizient erreichbar, wobei gleichzeitig eine ausreichende Stabilität der Gitterstruktur bereitgestellt wird.

Bei einer bevorzugten Ausgestaltung der erfindungsgemäßen Vorrichtung umfasst die Gitterstruktur mehrere erste Filamente und/oder mehrere zweite Filamente, wobei zumindest abschnittsweise die ersten Filamente jeweils den gleichen ersten Flechtwinkel α und die zweiten Filamente jeweils den gleichen zweiten Flechtwinkel β aufweisen. Konkret kann die Gitterstruktur durch wenigstens zwei Filamentgruppen gebildet sein, wobei die erste Filamentgruppe mehrere erste Filamente mit dem einheitlichen ersten Flechtwinkel α und die zweite Filamentgruppe mehrere zweite Filamente mit dem einheitlichen zweiten Flechtwinkel β aufweist. Innerhalb der Filamentgruppen verlaufen die Filamente jeweils parallel zueinander. Die Gitterstruktur kann insgesamt mehrere Filamente aufweisen, wobei in einem Axialabschnitt der Gitterstruktur jeweils höchstens zwei unterschiedliche Flechtwinkel gebildet sind. Ein Axialabschnitt der Gitterstruktur kann also höchstens zwei Filamentgruppen mit unterschiedlichen Flechtwinkeln umfassen. Die Anzahl der Filamente pro Drahtgruppe ist variabel. Die zwei Drahtgruppen mit unterschiedlichem Flechtwinkel können eine unterschiedliche Anzahl an Filamenten aufweisen.

Der erste Flechtwinkel α und/oder der zweite Flechtwinkel β können zumindest abschnittsweise entlang der Gitterstruktur variieren. In Längsrichtung der Gitterstruktur können also mehrere Axialabschnitte gebildet sein, die unterschiedliche Flechtwinkel α, β umfassen. Die Flechtwinkel können sich kontinuierlich entlang bzw. in Längsrichtung der Gitterstruktur ändern. Damit wird erreicht, dass sich entlang der Gitterstruktur unterschiedliche Torsionsgrade einstellen. Die Torsion der Gitterstruktur um die gemeinsame Rotationsachse kann also in Längsrichtung der Gitterstruktur unterschiedlich stark ausgeprägt sein. Bei einer bevorzugten Ausführungsform weist die Gitterstruktur wenigstens einen ersten Längsabschnitt und wenigstens einen zweiten Längsabschnitt auf, wobei die Flechtwinkel α, β im ersten Längsabschnitt und im zweiten Längsabschnitt eingestellt sind derart, dass der erste Längsabschnitt beim Übergang der Gitterstruktur vom radial komprimierten zum radial expandierten Zustand, oder umgekehrt, gegensinnig zum zweiten Längsabschnitt um die gemeinsame Rotationsachse verdreht wird. Durch die unterschiedlichen Torsionsrichtungen des ersten Längsabschnitts und des zweiten Längsabschnitts wird die Ablösung von Konkrementen aus Körperhohlräumen, insbesondere das Ablösen von Thromben bzw. Plaque-Partikeln von Gefäßwänden, erleichtert.

Vorzugsweise weisen das erste Filament und das zweite Filament im ersten Längsabschnitt und im zweiten Längsabschnitt jeweils unterschiedliche Abschnittslängen und über die gesamte Länge der Gitterstruktur im Wesentlichen die gleiche Gesamtlänge auf. Der erste Längsabschnitt und der zweite Längsabschnitt bilden jeweils Abschnitte der rotationssymmetrischen Gitterstruktur. Die Längsabschnitte weisen daher jeweils mehrere Filamente, insbesondere wenigstens ein erstes Filament und ein zweites Filament auf. Innerhalb der Längsabschnitte unterscheiden sich die einzelnen Filamente durch ihre Abschnittslänge, die jeweils entlang eines Filaments gemessen ist. Die Abschnittlänge und die Gesamtlänge eines Filaments beziehen sich also auf das Filament an sich. Die gesamte Länge der Gitterstruktur entspricht hingegen dem Abstand der axialen Enden der Gitterstruktur, gemessen entlang der gemeinsamen Rotationsachse. Da die innerhalb der Längsabschnitte der Gitterstruktur jeweils angeordneten Filamente zwar unterschiedliche Abschnittslängen aufweisen, die Gesamtlänge der einzelnen Filamente über die gesamte Länge der Gitterstruktur aber im Wesentlichen gleich ist, wird die Verdrehung der Verbindungsstellen zueinander im Wesentlichen ausgeglichen. Insbesondere wird auf diese Weise erreicht, dass sich die Torsionsgrade bzw. Torsionsgeschwindigkeiten des ersten Längsabschnitts und des zweiten Längsabschnitts derart relativ zueinander verhalten, dass die Lage der Verbindungsstellen am Ende der Zustandsänderung, beispielsweise nach erfolgter Expansion, gegenüber dem vorherigen Zustand, beispielsweise dem zuvor eingestellten komprimierten Zustand, unverändert ist. Die Verbindungsstellen nehmen somit ihre anfängliche Nulllage ein.

Mit anderen Worten ist es auch möglich, dass die Flechtwinkel α, β in unterschiedlichen Axialabschnitten der Gitterstruktur derart variieren, dass sich die Gesamtrotation der ersten Verbindungsstelle bezüglich der zweiten Verbindungsstelle aufhebt. Das bedeutet, dass beispielsweise in einem ersten Axialabschnitt eine Torsion der Gitterstruktur im Uhrzeigersinn, und in einem zweiten Axialabschnitt eine Torsion der Gitterstruktur entgegen dem Uhrzeigersinn erfolgt. Die Flechtwinkel in dem ersten und dem zweiten Längsabschnitt können dabei derart angepasst sein, dass sich die gesamte Torsion der beiden Längsabschnitte für einen bestimmten radialen Expansionsgrad derart eingestellt ist, dass die Verbindungsstellen nach vollständiger Expansion ihre ursprüngliche Nulllage einnehmen bzw. aufweisen.

Im Hinblick auf die relative Ortslage der Verbindungsstellen vor und nach der Durchmesseränderung bestehen im Wesentlichen zwei Möglichkeiten. Einerseits kann der Unterschied der Ortslagen gleich Null sein. Das bedeutet, dass die ursprüngliche Ortslage der Verbindungsstellen nach der Durchmesseränderung der Gitterstruktur wieder eingenommen wird. Änderungen der Ortslage der Verbindungsstellen während der Rotation werden ausgeglichen. Die Verbindungsstellen können die Nulllage mehrfach durchlaufen. Andererseits kann ein Unterschied bzw. eine Abweichung vorhanden sein, wobei die ursprüngliche Ortslage der Verbindungsstellen nach der Durchmesseränderung nicht wieder eingenommen wird.

Der vollständige Ausgleich, also die Einnahme der Nulllage, lässt sich auf verschiedene Weise verwirklichen. Die Gitterstrukturen der beiden Längsabschnitte können 1:1 gespiegelt sein. Die Flechtwinkel der beiden Gitterstrukturen sind dabei vertauscht bzw. punktsymmetrisch gespiegelt. Beispielsweise weist.der erste Längsabschnitt einen Flechtwinkel α₁ und einen Flechtwinkel β₁ und der zweite Längsabschnitt einen Flechtwinkel α₂ und einen Flechtwinkel β₂ auf, wobei α₁ = β₂ und β₁ = α₂. Konkret kann der erste Längsabschnitt eine Gruppe erster Filamente mit dem gleichen ersten Flechtwinkel α₁ und eine Gruppe zweiter Filamente mit dem gleichen zweiten Flechtwinkel β₁ aufweisen. Der zweite Längsabschnitt kann hingegen eine Gruppe erster Filamente mit dem gleichen ersten Flechtwinkel α₂ und eine Gruppe zweiter Filamente mit dem gleichen zweiten Flechtwinkel β₂ umfassen. Die ersten und zweiten Flechtwinkel α₁, β₁, α₂, β₂ in den beiden Längsabschnitten sind jeweils innerhalb eines Längsabschnitts unterschiedlich, so dass sich in den Längsabschnitten jeweils eine asymmetrische Struktur bzw. Konfiguration der Filamente einstellt. Alternativ oder zusätzlich kann der vollständige Ausgleich dadurch erreicht werden, dass die Länge der Längsabschnitte der Gitterstruktur, gemessen entlang der gemeinsamen Rotationsachse, identisch ist.

Es ist auch möglich, die jeweiligen Flechtwinkel der Längsabschnitte unterschiedlich auszubilden und den Ausgleich der Ortslage der Verbindungsstellen über die Länge der beiden Längsabschnitte zu steuern. Beispielsweise können α₁ ≠ β₂ und β₁ ≠ α₂ sein. Die Länge des ersten Längsabschnitts ist dabei ungleich der Länge des zweiten Längsabschnitts. Der erste Längsabschnitt kann länger ausgebildet sein als der zweite Längsabschnitt, wobei der Unterschied zwischen α₁ und β₁ relativ klein ist, so dass im ersten Längsabschnitt eine langsame Torsionsgeschwindigkeit eingestellt ist. Im zweiten Längsabschnitt ist die Steigung bzw. der Unterschied zwischen α₂ und β₂ größer als im ersten Längsabschnitt, so dass die Torsionsgeschwindigkeit des zweiten Längsabschnitts gegenüber dem ersten Längsabschnitt erhöht ist. Insgesamt gleicht sich dadurch der Verdrehwinkel zwischen den Verbindungsstellen aus.

Im Allgemeinen kann die Variation der Flechtwinkel entlang der Gitterstruktur fließend bzw. kontinuierlich verlaufen. Stufenweise bzw. diskrete Flechtwinkeländerungen entlang der Gitterstruktur sind ebenfalls möglich.

Eine Verbindungsstelle kann gegenüber der anderen Verbindungsstelle während des Übergangs der Gitterstruktur vom komprimierten in den expandierten Zustand, oder umgekehrt, zunächst in eine Richtung und im weiteren Verlauf in die Gegenrichtung rotieren, so dass die Relativposition der Verbindungsstellen vor und nach der Zustandsänderung der Gitterstruktur gleich ist. Es ist auch möglich, dass die Nulllage der Verbindungsstellen nach der Expansion durch eine mehrfache, vollständige Verdrehung der zweiten Verbindungsstelle gegenüber der ersten Verbindungsstelle eingestellt ist. Beispielsweise kann sich die zweite Verbindungsstelle gegenüber der ersten Verbindungsstelle bei der Expansion wenigstens einmal, insbesondere wenigstens zweimal, insbesondere wenigstens dreimal, insbesondere wenigstens viermal, vollständig, d.h. um 360°, um die gemeinsame Rotationsachse drehen, so dass nach Abschluss der Expansion beide Verbindungsstellen ihre ursprüngliche Position, insbesondere Nulllage bzw. Nullposition, aufweisen. Bei bzw. während der Expansion oder Komprimierung der Gitterstruktur rotiert bzw. tordiert die Gitterstruktur also zunächst in einer Richtung und ändert im weiteren Verlauf die Rotations- bzw. Torsionsrichtung. Generell erfolgt bei der Expansion bzw. allgemein bei der Zustandsänderung der Gitterstruktur eine Torsion der Gitterstruktur oder der Längsabschnitte der Gitterstruktur. Auch nach der Expansion ist die Gitterstruktur gegenüber der ursprünglichen Konfiguration verdreht bzw. tordiert. Die Verbindungsstellen können jedoch in die Nulllage zurückgeführt sein.

In einer besonders bevorzugten Ausführungsform weist die Gitterstruktur wenigstens einen ersten Längsabschnitt und wenigstens einen zweiten Längsabschnitt auf, wobei der erste Flechtwinkel α im ersten Längsabschnitt größer und im zweiten Längsabschnitt kleiner als der zweite Flechtwinkel β ist. Das hat den Vorteil, dass der erste Längsabschnitt und der zweite Längsabschnitt gegenläufig zueinander tordieren, wenn die Gitterstruktur vom expandierten in den komprimierten bzw. vom komprimierten in den expandierten Zustand überführt wird. Wenn der erste Flechtwinkel α im ersten Längsabschnitt gegenüber dem zweiten Flechtwinkel β im zweiten Längsabschnitt verschieden ist, ergibt sich zudem eine unterschiedliche Torsionsgeschwindigkeit der beiden Längsabschnitte zueinander. In dem speziellen Fall, dass der erste Flechtwinkel α im ersten Längsabschnitt gleich groß wie der zweite Flechtwinkel β im zweiten Längsabschnitt ist, und umgekehrt, ergibt sich eine gegenläufige Torsion der beiden Längsabschnitte derart, dass die Torsion der gesamten Gitterstruktur nach Abschluss der Zustandsänderung, beispielsweise nach erfolgter Expansion, im Wesentlichen Null ist. Die Torsionsgrade der beiden Längsabschnitte heben sich also auf, wenn das Winkelverhältnis zwischen den beiden Längsabschnitte umgekehrt ist.

Bei einer weiteren bevorzugten Ausführungsform der medizinischen Vorrichtung weisen die Filamente zumindest abschnittsweise wenigstens einen Schneidebereich, insbesondere eine Schneidefläche oder eine Schneidekante, auf. Der Schneidebereich begünstigt das Ablösen von Konkrementen aus Körperhohlräumen, so dass die Effizienz der Behandlung mit medizinischen Vorrichtungen erhöht wird.

Die Gitterstruktur kann im Bereich des ersten axialen Endes, insbesondere im Bereich des proximalen Endes bezogen auf den Standpunkt des Anwenders, einen trichterförmigen Abschnitt aufweisen. Der trichterförmige Abschnitt kann vorteilhaft eine Führungsfunktion bieten, so dass die Gitterstruktur durch einfache axiale Verschiebung in proximaler Richtung in ein Zufuhrsystem, insbesondere einen Katheter, komprimierbar ist. Die Vorrichtung kann auf diese Weise besonders einfach vom Behandlungsort entfernt werden. Es ist auch möglich, dass der trichterförmige Abschnitt oder ein weiterer trichterförmiger Abschnitt am zweiten axialen Ende, insbesondere an einem distalen Ende, der Gitterstruktur angeordnet ist. Der am zweiten axialen Ende angeordnete trichterförmige Abschnitt kann insbesondere als Filter dienen, der ein Abströmen von abgelösten Partikeln verhindert.

Die Gitterstruktur, insbesondere der trichterförmige Abschnitt, kann wenigstens abschnittsweise eine fluiddichte Abdeckung aufweisen. Es ist nicht ausgeschlossen, dass die fluiddichte Abdeckung eine Strukturierung, insbesondere eine Perforierung, umfasst. Die fluiddichte Abdeckung kann beispielsweise die Funktion eines Filters, insbesondere eines Partikelfilters, übernehmen. Ferner kann durch die fluiddichte Abdeckung ein Behandlungsraum, der unter anderem durch die Gitterstruktur aufgespannt wird, fluiddicht begrenzen. Das bietet die Möglichkeit, die Gitterstruktur mit einer Absaugvorrichtung zu kombinieren, die von dem Körperhohlraum gelöste Konkrementpartikel durch Erzeugung eines Unterdrucks aus dem Körperhohlraum entfernt bzw. absaugt. Durch die fluiddichte Abdeckung wird vermieden, dass durch den erzeugten Unterdruck Körperflüssigkeit aus Bereichen des Körperhohlraums entnommen wird, die außerhalb des Behandlungsortes angeordnet sind.

Bei einer zweckmäßigen Ausführungsform der Vorrichtung sind eine erste Gitterstruktur und wenigstens eine zweiten Gitterstruktur vorgesehen, die koaxial innerhalb der ersten Gitterstruktur angeordnet ist, wobei die Filamente der ersten Gitterstruktur gegenüber den Filamenten der zweiten Gitterstruktur unterschiedliche Flechtwinkel α, β, α', β' aufweisen derart, dass beim Übergang der Gitterstrukturen vom radial komprimierten zum radial expandierten Zustand, oder umgekehrt, die erste Gitterstruktur und die zweite Gitterstruktur relativ zueinander verdrehbar sind. Diese Ausführungsform kombiniert also zwei erfindungsgemäß ausgestaltete Gitterstrukturen, die jeweils für sich eine Rotation um die Längsachse bei einer Durchmesseränderung vollziehen. Dabei handelt es sich insbesondere um eine stent-in-stent Anordnung, die wenigstens zwei Stents umfasst. Da die Gitterstrukturen koaxial, bzw. konzentrisch ineinander angeordnet sind, kommt es zu einer Relativbewegung zwischen den beiden Gitterstrukturen. Durch die Relativbewegung werden radial nach innen durch die Zellen bzw. Maschen der Gitterstrukturen ragende Thrombenteile bzw. Konkrementteile abgeschert, die dann entfernt werden können. Dabei können Thrombenteile getrennt und einzeln eingefangen werden. Thrombenteile oder Thromben können sind auch zwischen den beiden Gitterstrukturen verfangen, ohne dass diese abgetrennt werden. Der Thrombus ist in der Vorrichtung verankert und kann durch zusammen mit der Vorrichtung bewegt und gelöst werden.

Die Relativbewegung kann bspw. durch Rotationen der beiden Gitterstrukturen in entgegensetzten Richtungen, also gegensinnig erfolgen. Die Flechtwinkel der Gitterstrukturen können so ausgebildet sein, dass eine der Gitterstrukturen stillsteht und nur die andere Gitterstruktur, insbesondere die innere oder äußere Gitterstruktur, rotiert. Es ist auch möglich, dass die Gitterstrukturen in der selben Richtung mit unterschiedlichen Geschwindigkeiten rotieren.

Bei einer weiteren bevorzugten Ausführungsform weisen die ersten Gitterstruktur und die zweite Gitterstruktur jeweils wenigstens zwei gleichsinnig um die gemeinsame Rotationsachse gewundene Filamente aufweisen, wobei die Filamente der ersten Gitterstruktur zumindest am gemeinsamen zweiten axialen Ende der Gitterstrukturen gegenüber den Filamenten der zweiten Gitterstruktur gegensinnig um die gemeinsame Rotationsachse gewunden sind, um einen scherenartig bzw. zangenartig betätigbaren Greifabschnitt zu bilden. Der am axialen Ende gebildete Greifabschnitt ist durch eine Durchmesseränderung betätigbar derart, dass der Greifabschnitt bei einer Durchmesseränderung in die eine Richtung sich öffnet und bei einer Durchmesseränderung in die andere Richtung sich schließt. Bspw. kann der Greifabschnitt durch eine Durchmesservergrößerung geöffnet und durch eine Durchmesserverkleinerung geschlossen werden. Die Bewegungen des Greifabschnitts können auch umgekehrt initiiert werden.

Die Relativbewegung der konzentrisch angeordneten Gitterstrukturen bzw der Komponenten des Greifabschnitts wird durch eine Längsverschiebung der Vorrichtung bezogen auf ein Zufuhrsystem erreicht. Durch die Längsverschiebung werden die Gitterstrukturen aus dem Zufuhrsystem entlassen beziehungsweise in das Zufuhrsystem eingezogen. Dadurch kommt es zu der gewünschten Durchmesseränderung, da die Gitterstrukturen durch die Längsverschiebung komprimiert bzw. expandiert werden.

Die Greifbewegung des Greifabschnitts kann zum Greifen und Lösen von Konkrementteilen oder anderen Teilen genutzt werden.
Ferner wird eine medizinische Vorrichtung zum Lösen von Konkrementen in Körperhohlorganen mit wenigstens einer rotationssymmetrischen Gitterstruktur offenbart, die mit einem Halteelement drehfest verbunden ist und wenigstens zwei um eine gemeinsame Rotationsachse L spiralförmig angeordnete und sich kreuzende Gitterstege aufweist, die an wenigstens zwei in Längsrichtung der Gitterstruktur beabstandet angeordneten Verbindungsstellen miteinander verbunden sind, wobei ein erster Gittersteg einen ersten Stegwinkel α und ein zweiter Gittersteg einen zweiten Stegwinkel β bildet, der zumindest abschnittsweise von dem ersten Stegwinkel α verschieden ist derart, dass eine erste Verbindungsstelle beim Übergang der Gitterstruktur von einem radial komprimierten in einen radial expandierten Zustand, oder umgekehrt, gegenüber einer zweiten Verbindungsstelle um die gemeinsame Rotationsachse L verdreht wird. Damit wird die Erfindung allgemein im Zusammenhang mit stegförmig aufgebauten Gitterstrukturen offenbart und beansprucht, die beispielsweise durch Schneiden, insbesondere durch Laserschneiden und/oder durch PVD-Verfahren und/oder durch Ätzverfahren hergestellt sind. Die vorstehend genannten Stegwinkel entsprechen den beim Flechten eingestellten Flechtwinkeln. Die Beschreibung der Flechtwinkel wird auch im Zusammenhang mit den Stegwinkeln offenbart.

Gemäß einem nebengeordneten Aspekt der Erfindung wird ein Verfahren zum Herstellen der zuvor beschriebenen medizinischen Vorrichtung beansprucht, das die folgenden Schritte umfasst:
- Bereitstellen einer Flechtmaschine, die um eine gemeinsame Rotationsachse rotierende Klöppel aufweist;
- Verbinden des ersten Filaments (11a) wird mit einem ersten Klöppel und des zweiten Filaments (11b) mit einem zweiten Klöppel;
- Einstellen der Rotation der Klöppel derart, dass das erste Filament (11a) mit dem erste Flechtwinkel α und das zweite Filament (11b) mit dem zweiten Flechtwinkel β zur Bildung der rotationssymmetrischen Gitterstruktur (10) gegensinnig um die gemeinsame Rotationsachse gewickelt werden;
- Verbinden des ersten Filaments (11a) mit dem zweiten Filament (11b) an der ersten Verbindungsstelle (12a) und an der von der ersten Verbindungsstelle (12a) beabstandet angeordneten zweiten Verbindungsstelle (12b)
   oder
   Umlenken eines gemeinsamen Drahtelements (11), das das erste Filament (11a) und das zweite Filament (11b) bildet, zur Bildung der ersten Verbindungsstelle (12a) und Verbinden von jeweils einem freien Ende des ersten Filaments (11a) mit einem freien Ende des zweiten Filaments (11b) zur Bildung der von der ersten Verbindungsstelle (12a) beabstandet angeordneten zweiten Verbindungsstelle (12b);
- Verbinden eines ersten axialen Endes (13a), insbesondere eines proximalen Endes, der Gitterstruktur (10) mit dem Halteelement (20).

Die einzelnen Verfahrensschritte des erfindungsgemäßen Herstellungsverfahrens können in ihrer zeitlichen Abfolge variieren. Insbesondere ist es vorteilhaft, das Umlenken des gemeinsamen Drahtelements zur Bildung der beiden Filamente vor dem Flechten der Gitterstruktur durchzuführen. Ferner können die beiden Filamente vor dem Flechten an wenigstens einer, insbesondere der ersten, Verbindungsstelle miteinander verbunden werden. Die zweite Verbindungsstelle kann nach dem Flechten erstellt werden.

Ein weiterer nebengeordneter Aspekt der Erfindung betrifft ein Behandlungssystem mit einer zuvor beschriebenen medizinischen Vorrichtung und einem Katheter, in den das Halteelement längsverschiebbar angeordnet ist. Das Halteelement ist mit einem ersten axialen Ende der Gitterstruktur verbunden, das einem proximalen Ende des Katheters zugewandt ist.

Das im Rahmen der Erfindung ferner offenbarte und beanspruchte Verfahren zum Herstellen eines Behandlungssystems, insbesondere eines zuvor beschriebenen Behandlungssystems, umfasst die folgenden Schritte:
- Bereitstellen einer Schleuse mit einem proximalen Ende und einem distalen Ende;
- Anordnen des Halteelements (20) in der Schleuse, wobei das Halteelement (20) vom distalen Ende zum proximalen Ende der Schleuse geführt wird;
- Überführen der Gitterstruktur (10) von einem radial expandierten in einen radial komprimierten Zustand durch Einziehen der Gitterstruktur (10) in das distale Ende der Schleuse, wobei die zweite, insbesondere an einem zweiten, freien axialen Ende (13b) der Gitterstruktur (10) angeordnete, Verbindungsstelle (12b) der Gitterstege, insbesondere Filamente (11a, 11b) relativ zum Halteelement (20) um die gemeinsame Rotationsachse verdreht wird.

Die Vorrichtung gemäß Anspruch 1 sowie alle vorstehend beschriebenen Ausführungsformen und die nachfolgenden Ausführungsbeispiele werden sowohl für sich als auch zusammen mit einem Zufuhrsystem, insbesondere einem Katheter, in der Form einer Anordnung beschrieben und beansprucht. Dabei wirken die Vorrichtung und das Zufuhrsystem zusammen derart, dass durch zumindest teilweises Einziehen oder Entlassen der Durchmesser der Vorrichtung änderbar ist, so dass eine Rotation der Gitterstruktur erreicht wird. Bei diesem Vorgang tordiert der zumindest temporär im Gefäß befindlich expandierte Teil der Vorrichtung und führt die gewünscht Schabbeweggung aus. Es ist auch möglich, die Vorrichtung vollständig zu entlassen oder vollständig einzuziehen.

Die Erfindung wird im Folgenden anhand von Ausführungsbeispielen unter Bezugnahme auf die beigefügten, schematischen Zeichnungen näher erläutert. Darin zeigen
- Fig. 1: eine räumliche Ansicht einer erfindungsgemäßen medizinischen Vorrichtung gemäß einem bevorzugten Ausführungsbeispiel;
- Fig. 2: eine räumliche Ansicht einer erfindungsgemäßen medizinischen Vorrichtung gemäß einem weiteren bevorzugten Ausführungsbeispiel;
- Fig. 3: eine Seitenansicht der medizinischen Vorrichtung gemäß Fig. 2;
- Fig. 4a, 4b: eine schematische Darstellung der trigonometrischen Zusammenhänge, die sich beim Übergang der Gitterstruktur der medizinischen Vorrichtung vom expandierten in den komprimierten Zustand einstellen;
- Fig. 5: eine schematische Darstellung einer Gitterstruktur mit einer asymmetrischen Gitterkonfiguration gemäß einem bevorzugten Ausführungsbeispiel;
- Fig. 6: eine schematische Darstellung der Anordnung zweiter Filamente in unterschiedlichen Längsabschnitten der Gitterstruktur gemäß einem bevorzugten Ausführungsbeispiel;
- Fig. 7: eine schematische Darstellung der Anordnung der Komponenten eines Greifabschnitts, der durch die Torsionswirkung betätigbar ist,
- Fig. 8: eine räumliche Ansicht einer medizinischen Vorrichtung nach einem weiteren erfindungsgemäßen Ausführungsbeispiel im expandierten Zustand, bei dem zwei Gitterstrukturen konzentrisch angeordnet sind, und
- Fig. 9: eine räumliche Ansicht der medizinischen Vorrichtung gemäß Fig. 8 im teilkomprimierten Zustand.

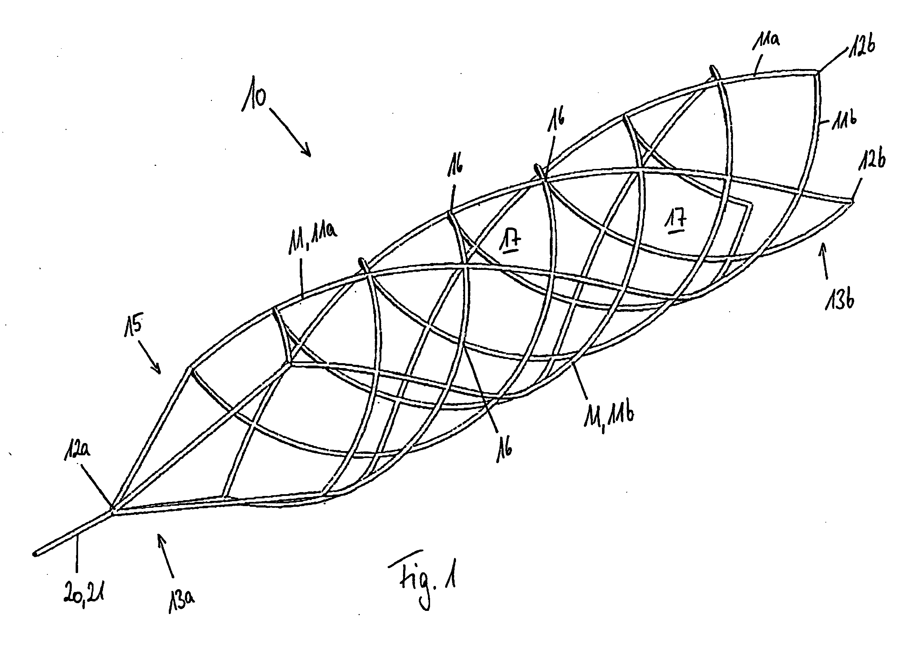

Fig. 1 zeigt eine Gitterstruktur 10, die mehrere Drahtelemente 11 umfasst. Die Drahtelemente 11 bilden eine netzartige bzw. gitterartige Struktur. Die Gitterstruktur 10 ist rotationssymmetrisch, insbesondere hohlzylindrisch ausgebildet. Die Drahtelemente 11 sind zumindest abschnittsweise spiralförmig um eine gemeinsame Rotationsachse L angeordnet.

Die Gitterstruktur 10 weist ein erstes axiales Ende 13a und ein zweites axiales Ende 13b auf. Das erste axiale Ende 13a bzw. ein proximales Ende der Gitterstruktur 10 ist mit einem Halteelement 20 verbunden. Das Halteelement 20 kann einen Führungsdraht 21 umfassen bzw. einen Führungsdraht 21 bilden. Das Halteelement 20 kann auch ein Verbindungsglied zwischen dem Führungsdraht 21 und der Gitterstruktur 10 bilden. Es ist auch möglich, dass das Haltemittel 20 ein Verbindungsglied zwischen der Gitterstruktur 10 und einem Katheter bzw. allgemein einem Zufuhrsystem bildet.
Die Drahtelemente 11 bilden mehrere Filamente 11a, 11b. Ein erstes Filament 11a ist ausgehend vom ersten axialen Ende 13a der Gitterstruktur 10 spiralförmig im Uhrzeigersinn um die gemeinsame Rotationsachse L gewunden. Das zweite Filament 11b ist gegensinnig, also ausgehend vom ersten axialen Ende 13a entgegen dem Uhrzeigersinn, spiralförmig entlang der gemeinsamen Rotationsachse L gewunden. Die Filamente 11a, 11b kreuzen sich an mehreren Kreuzungspunkte 16 der Gitterstruktur 10. In den Kreuzungspunkten 16 sind die Filamente 11a, 11b zueinander frei beweglich. Die medizinische Vorrichtung gemäß Fig. 1 weist mehrere, insbesondere vier, erste Filamente 11a und mehrere, insbesondere vier, zweite Filamente 11b auf. Eine andere Anzahl von Filamenten 11a, 11b, ist möglich.

Die ersten Filamente 11a verlaufen zueinander im Wesentlichen parallel. Das bedeutet, dass die ersten Filamente 11a jeweils den gleichen ersten Flechtwinkel α bilden. Der Flechtwinkel wird dabei als der Winkel bestimmt, der sich zwischen einer Projektion der Rotationsachse L auf die Umfangsebene der Gitterstruktur 10 und dem ersten oder zweiten Filament 11a, 11b ausbildet. Analog verlaufen die zweiten Filamente 11b untereinander parallel bzw. bilden den gleichen zweiten Flechtwinkel β. Der erste Flechtwinkel α und der zweite Flechtwinkel β unterscheiden sich. Die ersten Filamente 11a weisen also gegenüber den zweiten Filamenten 11b einen anderen Flechtwinkel α, β auf. In Fig. 1 ist gut zu erkennen, dass die ersten Filamente 11a um die gemeinsame Rotationsachse L gewundene Spiralen bilden, die untereinander im Wesentlichen die gleiche Steigung aufweisen. Die zweiten Filamente 11b bilden gegenläufig um die gemeinsame Rotationsachse L gewundene Spiralen, die untereinander ebenfalls die gleiche Steigung aufweisen. Alle ersten Filamente 11a weisen gegenüber allen zweiten Filamenten 11b jedoch eine unterschiedliche Steigung auf. Insbesondere bilden die ersten Filamente 11a gemäß Fig. 1 eine flachere Steigung bzw. einen kleineren ersten Flechtwinkel α als alle zweiten Filamente 11b, die eine vergleichsweise steilere Steigung bzw. einen vergleichsweise größeren Flechtwinkel β bilden.

Die Filamente 11a, 11b können, wie in Fig. 1 dargestellt, miteinander verflochten sein. Dabei verläuft ein erstes Filament 11a abwechselnd über bzw. unter einem zweiten Filament 11b. Eine Flechtart, bei der das erste Filament 11a ein zweites Filament 11b abwechselnd überkreuzt und unterkreuzt, wird 1-über-1-Flechtung genannt. Andere Flechtarten sind möglich. Beispielsweise kann eine 1-über-2-Flechtung, 1-über-3-Flechtung, 1-über-4-Flechtung, 2-über-3-Flechtung, 2-über-4-Flechtung oder dergleichen vorgesehen sein. Ferner ist es möglich, dass die ersten und zweiten Filamente 11a, 11b jeweils Spiralen bilden, die vollständig ineinander angeordnet sind bzw. eine Spirale-in-Spirale-Konfiguration aufweisen. Das bedeutet, dass das erste Filament 11a beispielsweise die zweiten Filamente 11b generell überkreuzt. Alternativ kann das erste Filament 11a das zweite Filament 11b generell unterkreuzen. Überdies ist nicht ausgeschlossen, dass die Gitterstruktur 10 eine lasergeschnittene Struktur umfasst. Die Herstellung der Gitterstruktur 10 aus geflochtenen oder zumindest sich überkreuzenden Drahtelementen ist bevorzugt.

Die Gitterstruktur 10 weist wenigstens zwei Verbindungsstellen 12a, 12b auf, in denen die Filamente 11a, 11b miteinander verbunden sind. Bei dem Ausführungsbeispiel gemäß Fig. 1 sind die Verbindungsstellen 12a, 12b an den axialen Enden 13a, 13b der Gitterstruktur 10 angeordnet. Eine andere Position der Verbindungsstellen 12a, 12b ist möglich. Beispielsweise können die Verbindungsstellen 12a, 12b oder zumindest eine der Verbindungsstellen 12a, 12b in einzelnen Kreuzungspunkten 16 der Gitterstruktur 10 angeordnet sein.

Konkret umfasst die Gitterstruktur 10 eine erste Verbindungsstelle 12a, die am ersten axialen Ende 13a bzw. am proximalen Ende der Gitterstruktur 10 angeordnet ist. An der ersten Verbindungsstelle 12a sind alle Filamente 11a, 11b zusammengeführt und miteinander gekoppelt. Die erste Verbindungsstelle 12a ist auf der Rotationsachse L der Gitterstruktur 10 angeordnet. Eine andere Anordnung der ersten Verbindungsstelle 12a ist möglich. Von der ersten Verbindungsstelle 12a erstreckt sind in proximaler Richtung ferner das Halteelement 20. Das Halteelement 20 kann auch zumindest teilweise durch die Filamente 11a, 11b bzw. allgemein die Drahtelemente 11 der Gitterstruktur 10 gebildet sein. Beispielsweise können die Drahtelemente 11 der Gitterstruktur 10 am ersten axialen Ende 13a zusammengeführt und im Bereich der Verbindungsstelle 12a miteinander verbunden, insbesondere verdrillt oder eng verflochten sein. Die verdrillten bzw. verflochtenen Drahtelemente 11 können sich als Haltemittel 20 in proximaler Richtung fortsetzen.

Die zweite Verbindungsstelle 12b ist bei dem Ausführungsbeispiel gemäß Fig. 1 am zweiten axialen Ende bzw. am distalen Ende der Gitterstruktur 10 angeordnet. Insbesondere sind mehrere zweite Verbindungsstellen 12b vorgesehen, die jeweils ein erstes Filament 11a und ein zweites Filament 11b verbinden. Bei dem Ausführungsbeispiel gemäß Fig. 1 sind konkret vier zweite Verbindungsstellen 12b vorgesehen. Im Allgemeinen kann die Verbindung zwischen dem Filamenten 11a, 11b kraft- und/oder formschlüssig, beispielsweise durch Verdrillen, eng Verflechten und/oder Verkleben, erfolgen. Eine stoffschlüssige Verbindung, beispielsweise Schweißen oder Löten, ist ebenfalls möglich. Alternativ können das erste Filament 11a und das zweite Filament 11b, wie in Fig. 1 vorgesehen, aus demselben Drahtelement 11 gebildet sein. Das Drahtelement 11 ist am zweiten axialen Ende 13b der Gitterstruktur 10 umgelenkt bzw. umgebogen und bildet somit die zweite Verbindungsstelle 12b. Von der zweiten Verbindungsstelle 12b bzw. der Umlenkstelle des Drahtelements 11 aus erstreckt sich im Uhrzeigersinn spiralförmig um die Rotationsachse L das erste Filament 11a und im Gegenuhrzeigersinn entlang der Rotationsachse L das zweite Filament 11b. Die Filamente 11a, 11b sind an der zweiten Verbindungsstelle 12b einstückig miteinander verbunden.

Zwischen den Verbindungsstellen 12a, 12b bildet die rotationssymmetrische Gitterstruktur 10 ein asymmetrisches Geflecht. Das bedeutet, dass die Filamente 11a, 11b zwischen den Verbindungsstellen 12, 12b unterschiedliche Flechtwinkel α, β bzw. unterschiedliche Steigungen aufweisen. Aufgrund der unterschiedlichen Flechtwinkel α, β bzw. der unterschiedlichen Spiralsteigungen weisen die Filamente 11a, 11b unterschiedliche Filamentlängen auf. Konkret weisen die zweiten Filamente 11b gemäß Fig. 1 jeweils eine größere Filamentlänge als die ersten Filamente 11a auf. Da die Filamente 11a, 11b an den Verbindungsstellen 12a, 12b miteinander gekoppelt sind, wird beim Übergang der Gitterstruktur 10 von einem radial komprimierten in einen radial expandierten Zustand, oder umgekehrt, eine Rotation der Gitterstruktur 10 zwischen den Verbindungsstellen 12a, 12b bewirkt. Insbesondere verdreht sich bei der Expansion der Gitterstruktur die zweite Verbindungsstelle 12b gegenüber der ersten Verbindungsstelle 12a.

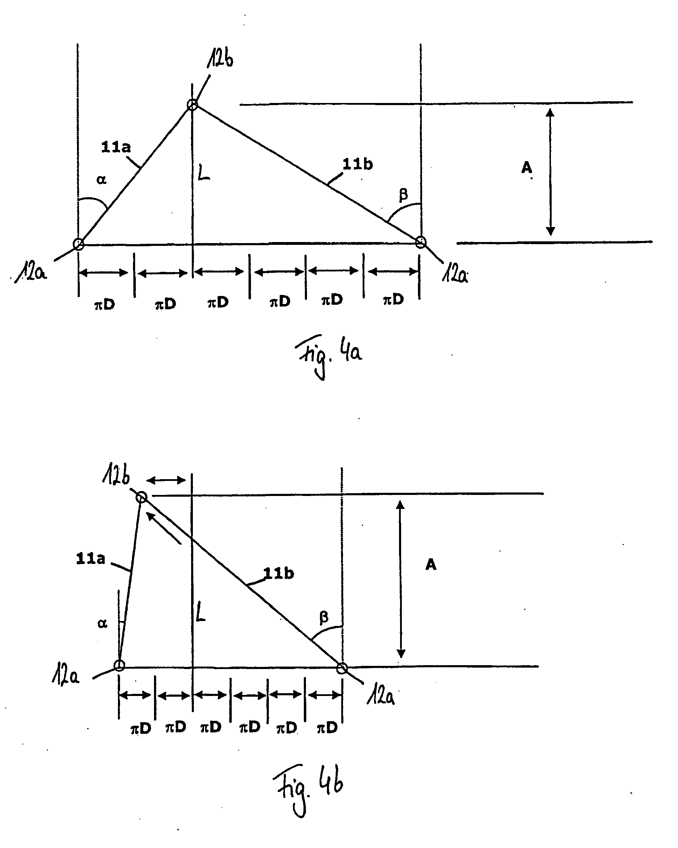

Der Rotationseffekt beim Übergang der Gitterstruktur 10 von einem radial expandierten Zustand zu einem radial komprimierten Zustand ist schematisch in den Fig. 4a, 4b verdeutlicht. In den Fig. 4a, 4b sind ein erstes Filament 11a und ein zweites Filament 11b dargestellt, die an der zweiten Verbindungsstelle 12b miteinander verbunden sind. In der rotationssymmetrischen Gitterstruktur 10 sind die Filamente 11a, 11b spiralförmig um die gemeinsame Rotationsachse L gewunden. Um die trigonometrischen Verhältnisse bei der Zustandsänderung der Gitterstruktur 10 darzustellen, sind die Filamente 11a, 11b in den Fig. 4a, 4b abgewickelt gezeigt. Das erste Filament 11a ist zweimal um die gemeinsame Rotationsachse L gewunden, wie durch die doppelte Angabe des Umfangs πD gezeigt. Das zweite Filament 11b ist demgegenüber viermal um die gemeinsame Rotationsachse L gewickelt (viermal πD). Das erste Filament 11a und das zweite Filament 11b sind an der ersten Verbindungsstelle 12a miteinander verbunden. Zur Betrachtung der trigonometrischen Verhältnismäßigkeiten ist die erste Verbindungsstelle 12a zweifach dargestellt. In dreidimensionaler Anordnung der Filamente 11a, 11b bildet die zweite Verbindungsstelle 12a einen einzigen Punkt der Gitterstruktur 10.

Wie in Fig. 4a gut zu erkennen, weist das zweite Filament 11b eine größere Abschnittslänge zwischen der ersten Verbindungsstelle 12a und der zweiten Verbindungsstelle 12b als das erste Filament 11a auf. Gleichzeitig bildet das zweite Filament 11 einen zweiten Flechtwinkel β, der größer als der erste Flechtwinkel α des ersten Filaments 11a ist. Der axiale Abstand der ersten Verbindungsstelle 12a von der zweiten Verbindungsstelle 12b wird in den Fig. 4a, 4b als Längserstreckung der Gitterstruktur 10 bzw. Abstand A der Verbindungsstellen 12a, 12b angegeben. Die zweite Verbindungsstelle 12b ist gemäß Fig. 4a auf der Rotationsachse L bzw. konkret auf einer Projektion der Rotationsachse L in die Umfangsebene der Gitterstruktur 10 angeordnet. Beim Übergang der Gitterstruktur 10 vom expandierten Zustand in den komprimierten Zustand ändert sich der Umfang πD der Gitterstruktur 10, wie in Fig. 4b gezeigt. Die Durchmesseränderung führt gleichzeitig zu einer Verlängerung bzw. Streckung der Gitterstruktur 10. Konkret wird der Abstand A zwischen der ersten Verbindungsstelle 12a und der zweiten Verbindungsstelle 12b vergrößert. Aufgrund der unterschiedlichen Flechtwinkel α, β bzw. der unterschiedlichen Abschnittslängen der Filamente 11a, 11b verschiebt sich die zweite Verbindungsstelle 12b aus ihrer ursprünglichen Lage bezüglich der Rotationsachse L. Konkret stellt sich zwischen der ersten Verbindungsstelle 12a und der zweiten Verbindungsstelle 12b ein Winkelversatz bezüglich der Rotationsachse L ein. Die Filamente 11a, 11b bzw. allgemein der Gitterstrukturabschnitt zwischen den Verbindungsstellen 12a, 12b verdreht sich bzw. tordiert um die Rotationsachse L. Die Bewegung des zweiten Verbindungspunkts 12b ist in Fig. 4b durch Pfeile angedeutet. Dabei ist zu erkennen, dass sich die Verbindungsstelle 12b sowohl in axialer Richtung, als auch in Umfangsrichtung aus der ursprünglichen Lage entfernt. Die Verschiebung der zweiten Verbindungsstelle 12b in Umfangsrichtung entspricht bei einer dreidimensionalen Struktur insbesondere der rotationssymmetrischen Gitterstruktur 10, einer Rotation.

Die Expansion der medizinischen Vorrichtung bzw. der Gitterstruktur 10 erfolgt vorzugsweise in einem Körperhohlraum. Dabei wird die Gitterstruktur 10 in distale Richtung, also in Richtung des zweiten axialen Endes 13b, aus einem Zufuhrsystem, beispielsweise einem Katheter, entlassen. Zwischen dem Katheter und der Gitterstruktur 10 erfolgt also eine Relativbewegung, wobei bevorzugt ist, dass die Gitterstruktur 10 ortsfest gehalten und der Katheter in proximaler Richtung gezogen wird. Die Gitterstruktur 10 umfasst vorzugsweise superelastische oder elastische Materialien, insbesondere Formgedächtnismaterialien, so dass die Expansion der Gitterstruktur 10 selbsttätig erfolgt. Konkret weitet sich also die Gitterstruktur 10 ausgehend vom zweiten axialen Ende 13b zum ersten axialen Ende 13a radial auf. Bei der radialen Aufweitung wird eine Verkürzung der durch die Filamente 11a, 11b gebildeten Spiralen bewirkt. Der Abstand A der Verbindungsstellen 12a, 12b verringert sich. Gleichzeitig wird durch die unterschiedlichen Flechtwinkel α, β eine Relativrotation zwischen den Verbindungsstellen 12a, 12b bewirkt. Dabei kann sich die Anzahl der Umwicklungen eines Filaments 11a, 11b um die gemeinsame Rotationsachse L ändern.

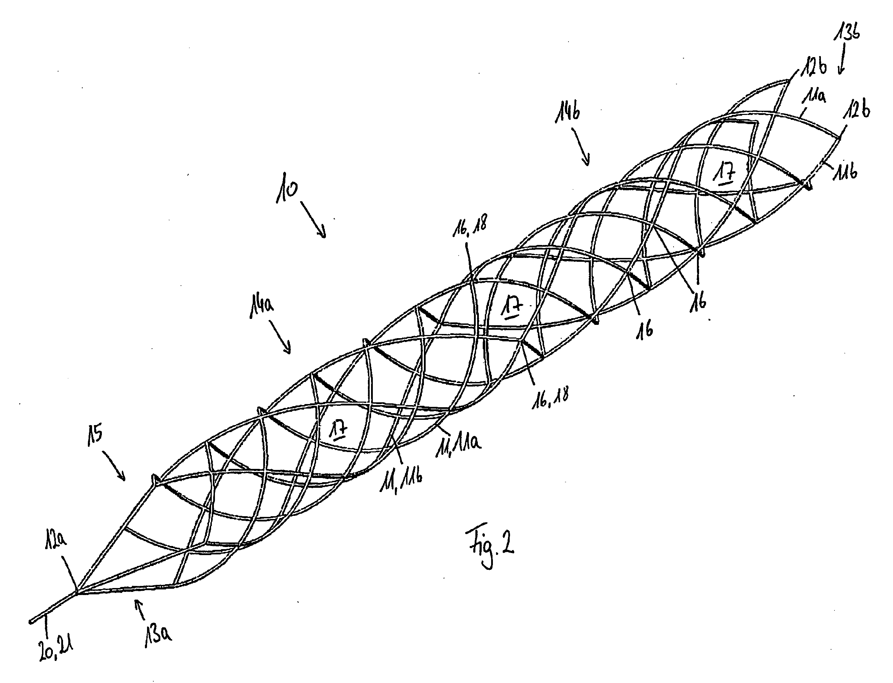

In Fig. 2 ist ein weiteres Ausführungsbeispiel der erfindungsgemäßen medizinischen Vorrichtung gezeigt, das sich von dem Ausführungsbeispiel gemäß Fig. 1 dadurch unterscheidet, dass die Gitterstruktur 10 zwei Längsabschnitte 14a, 14b umfasst, wobei die Filamente 11a, 11b in den Längsabschnitten 14a, 14b jeweils unterschiedliche Flechtwinkel aufweisen. Insbesondere unterscheiden sich die Längsabschnitte 14a, 14b durch einen unterschiedlichen Rotationsgrad voneinander. Dabei wird der Rotationsgrad als das Verhältnis zwischen der Rotation und der Änderung des Querschnittsdurchmessers der Gitterstruktur 10 bei der Expansion bzw. Komprimierung bezeichnet. Konkret weist die Gitterstruktur 10 gemäß Fig. 2 einen ersten Längsabschnitt 14a und einen zweiten Längsabschnitt 14b auf. Der erste Längsabschnitt 14a umfasst mehrere erste Filamente 11a, die einen ersten Flechtwinkel α bilden. Ferner sind mehrere zweite Filamente 11b vorgesehen, die einen zweiten Flechtwinkel β bilden. Der erste Flechtwinkel α und der zweite Flechtwinkel β unterscheiden sich. Der erste Längsabschnitt 14a ist durch eine erste Verbindungsstelle 12a, die am ersten axialen Ende der Gitterstruktur 10, insbesondere am proximalen Ende der Gitterstruktur 10, angeordnet ist, in proximaler Richtung begrenzt. Die erste Verbindungsstelle 12a verbindet die Drahtelemente 11, insbesondere die Filamente 11a, 11b, mit dem Halteelement 20. Die erste Verbindungsstelle 12a ist derart angeordnet, dass das Halteelement 20 koaxial zur Gitterstruktur 10 angeordnet ist. Eine andere Anordnung ist möglich. Beispielsweise kann das Halteelement 20 in der Umfangsebene der Gitterstruktur 10 angeordnet sein.

In distaler Richtung, insbesondere in Richtung des zweiten axialen Endes 13b der Gitterstruktur 10, ist der erste Längsabschnitt 14a durch mehrere Übergangsstellen 18 begrenzt. Die Übergangsstellen 18 sind jeweils in Kreuzungspunkten 16 der Filamente 11a, 11b angeordnet. Alternativ können die Übergangsstellen 18 auch jeweils zwischen zwei Kreuzungspunkten 16 angeordnet sein. Die Übergangsstellen 18 sind im Wesentlichen auf derselben Axialebene angeordnet, wobei sich die Axialebene senkrecht zur Rotationsachse L erstreckt. Mit anderen Worten sind die Übergangsstellen 18 auf der gleichen axialen Höhe der Gitterstruktur 10 angeordnet.

Die Übergangsstellen 18 begrenzen außerdem den zweiten Längsabschnitt 14b in proximaler Richtung. Der zweite Längsabschnitt 14b ist in distaler Richtung, also in Richtung des zweiten axialen Endes 13b der Gitterstruktur 10, durch mehrere zweite Verbindungsstellen 12b begrenzt. Die zweiten Verbindungsstellen 12b sind durch Umlenkstellen der Drahtelemente 11 gebildet. Die Filamente 11a, 11b sind also im Bereich der zweiten Verbindungsstellen 12b einstückig miteinander verbunden bzw. einstückig durch jeweils ein einziges Drahtelement 11 gebildet. Der zweite Längsabschnitt 14b umfasst analog zum ersten Längsabschnitt 14a mehrere erste Filamente 11a, die sich aus den ersten Filamenten 11a des ersten Längsabschnitts 14a fortsetzen und mehrere zweite Filamente 11b, die eine Fortsetzung der zweiten Filamente 11b des ersten Längsabschnitts 14a bilden. Die ersten Filamente 11a des zweiten Längsabschnitts 14b weisen einen ersten Flechtwinkel α, und die zweiten Filamente 11b einen zweiten Flechtwinkel β auf. Die Flechtwinkel α, β im zweiten Längsabschnitt 14b sind zueinander unterschiedlich.

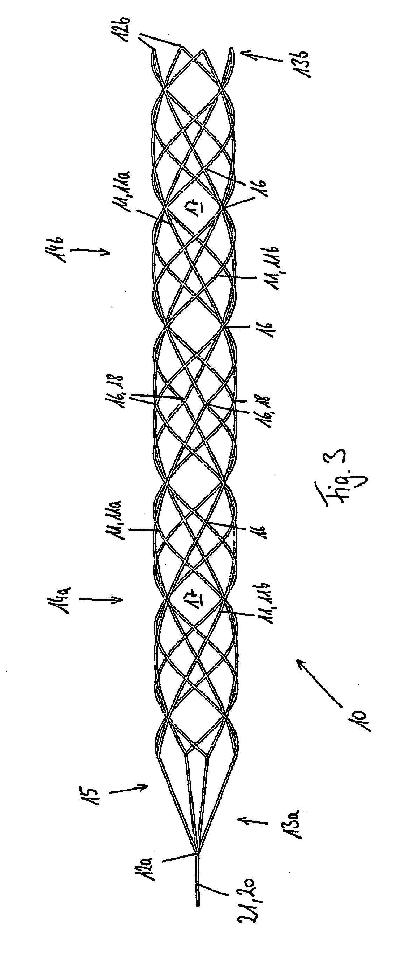

In Fig. 3 ist gut zu erkennen, dass sich die Flechtwinkel α, β an den Übergangsstellen 18 ändern. Das bedeutet, dass die ersten Filamente 11a im ersten Längsabschnitt 14a eine andere Steigung aufweisen als im zweiten Längsabschnitt 14b. Der erste Flechtwinkel α ändert sich im Bereich der Übergangsstelle 18, so dass im zweiten Längsabschnitt 14b ein anderer erster Flechtwinkel α als im ersten Längsabschnitt 14a gebildet ist. Dasselbe gilt für den zweiten Flechtwinkel β, der im zweiten Längsabschnitt 14b der Gitterstruktur 10 einen anderen Wert aufweist als im ersten Längsabschnitt 12a. Bei der Expansion der Gitterstruktur 10 rotiert die zweite Verbindungsstelle 12b gegenüber der Übergangsstelle 18 mit einem anderen Rotationsgrad bzw. einer anderen Rotationsgeschwindigkeit als die Übergangsstelle 18 gegenüber der ersten Verbindungsstelle 12a. Konkret erfährt der zweite Längsabschnitt 14b eine Torsion um die gemeinsame Rotationsachse L mit einer anderen Torsionsgeschwindigkeit als der erste Längsabschnitt 14a. Der Torsionsgrad bzw. die Torsionsgeschwindigkeit kann insbesondere durch entsprechende Wahl der unterschiedlichen Flechtwinkel α, β eingestellt werden. Dabei können die Flechtwinkel α, β derart eingestellt sein, dass der zweite Längsabschnitt 14b gegenüber dem ersten Längsabschnitt 14a gegensinnig tordiert bzw. rotiert. Die gegensinnige Torsion bzw. Rotation kann überdies derart angepasst sein, dass sich die Torsionsgrade bzw. Torsionsgeschwindigkeiten aufheben. Insbesondere kann der Torsionsgrad des ersten Längsabschnitts 14a gleich dem Torsionsgrad des zweiten Längsabschnitts 14b gleich sein, wobei die Torsionsrichtung umgekehrt ist, so dass sich die Verdrehung der Verbindungsstellen 12a, 12b insgesamt nach Abschluss der Durchmesseränderung der Gitterstruktur 10 aufhebt.

Die Relativposition zwischen der zweiten Verbindungsstelle 12b und der ersten Verbindungsstelle 12a kann auf diese Weise nach der Expansion gleich der Position vor der Expansion sein. Dazu ist besonders vorteilhaft vorgesehen, dass die ersten Filamente 11a gegenüber den zweiten Filamenten 11b abschnittsweise, beispielsweise im ersten und zweiten Längsabschnitt 14a, 14b, jeweils unterschiedliche Abschnittslängen aufweisen, wobei die Gesamtlänge der Filamente 11a, 11b über die gesamte Gitterstruktur 10 gleich ist. Konkret kann das erste Filament 11a im ersten Längsabschnitt 14a, also zwischen der ersten Verbindungsstelle 12a und der zweiten Verbindungsstelle 12b, kürzer als das zweite Filament 11b sein. Umgekehrt ist das erste Filament 11a im zweiten Längsabschnitt 14b länger als das zweite Filament 11b. Wenn das erste Filament 11a im zweiten Längsabschnitt 14b die gleiche Länge aufweist wie das zweite Filament 11b im ersten Längsabschnitt 14a und umgekehrt, gleichen sich die Filamentlängen über die gesamte Gitterstruktur 10 aus, so dass es zwar abschnittsweise innerhalb der Gitterstruktur zu einer Torsion kommt. Nach der Expansion der Gitterstruktur 10 weisen die Verbindungsstellen 12a, 12b jedoch die ursprüngliche, nichtrotierte Ursprungslage bzw. Nulllage auf.

Es ist grundsätzlich möglich, dass die Gitterstruktur 10 mehrere, insbesondere mehr als zwei, Längsabschnitte 14a, 14b aufweist. Der Ausgleich der Ortslage der Verbindungsstellen 12a, 12b kann über die gesamte Gitterstruktur 10 bzw. über alle Längsabschnitte 14a, 14b erfolgen. Die Summe aller Abschnittslängen der einzelnen Filamente 11a, 11b ist über die gesamte Gitterstruktur 10 gleich, um den Ausgleich zu erreichen.

Die einzelnen Längsabschnitte 14a, 14b umfassen vorzugsweise jeweils eine asymmetrische Konfiguration, d.h. einen Gitterstrukturabschnitt mit unterschiedlichen Flechtwinkeln α, β, auf. Es ist auch möglich, dass einzelne Längsabschnitte 14a, 14b eine symmetrische Konfiguration, also einen Gitterstrukturabschnitt mit unterschiedlichen Flechtwinkeln α, β, bilden. Im Allgemeinen lässt sich die asymmetrische Gitterstruktur 10 mit symmetrischen Strukturen bzw. Geflechten kombinieren.

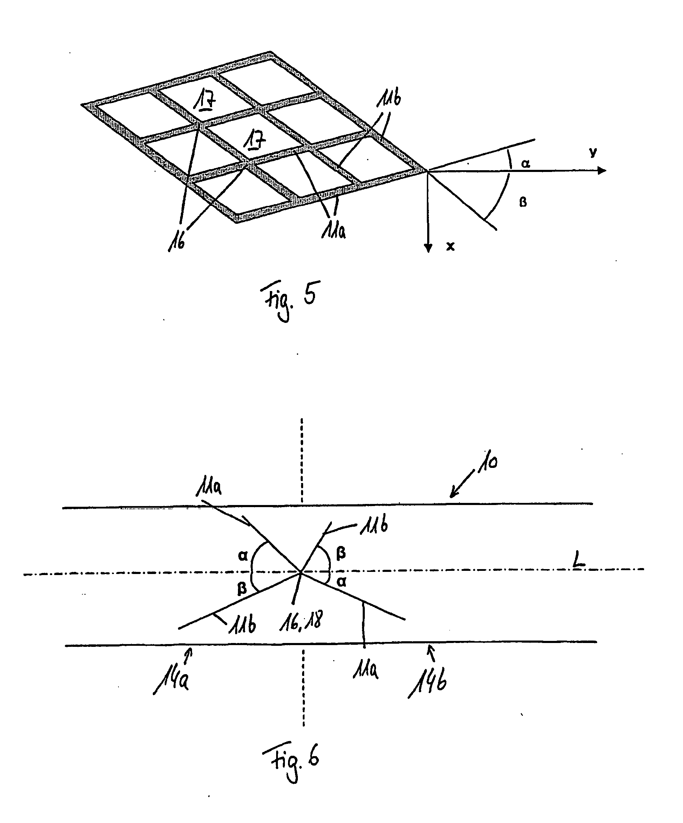

In den Fig. 5 und 6 ist die asymmetrische Struktur der Drahtelemente 11 bzw. der Filamente 11a, 11b, schematisch verdeutlicht. Fig. 5 zeigt einen Ausschnitt einer Gitterstruktur 10, die mehrere, sich kreuzende Filamente 11a, 11b umfasst. Die Filamente sind derart überkreuzt, dass sich zwischen den Filamenten 11a, 11b Maschen 17 bzw. Zellen bilden. In Axialrichtung y, also parallel zur Rotationsachse L, der Gitterstruktur betrachtet, weisen die Filamente 11a, 11b unterschiedliche Flechtwinkel α, β auf. Insbesondere bilden die ersten Filamente 11a einen ersten Flechtwinkel α, der bei dem Ausführungsbeispiel gemäß Fig. 5 kleiner als der zweite Flechtwinkel β der zweiten Filamente 11b ist.

In Fig. 6 ist der Übergang der Filamente 11a, 11b zwischen unterschiedlichen Längsabschnitten 14a, 14b der Gitterstruktur 10 schematisch verdeutlicht. Darin ist zu erkennen, dass im ersten Längsabschnitt 14a das erste Filament 11a einen ersten Flechtwinkel α bildet, der größer als der zweite Flechtwinkel β des zweiten Filaments 11b ist. Zwischen den Längsabschnitten 14a, 14b sind die Filamente 11a, 11b an einer zweiten Verbindungsstelle 12b miteinander verbunden. Im Bereich der zweiten Verbindungsstelle 12b ändern sich die Flechtwinkel α, β. Insbesondere weist das erste Filament 11a im zweiten Längsabschnitt 14b einen kleineren ersten Flechtwinkel α auf als im ersten Längsabschnitt 14a. Umgekehrt weist das zweite Filament 11b im zweiten Längsabschnitt 14b einen größeren zweiten Flechtwinkel β als im ersten Längsabschnitt 14a auf. Durch die unterschiedlichen Flechtwinkel zwischen den Längsabschnitten 14a, 14b bilden sich in den Längsabschnitten 14a, 14b unterschiedliche Torsionsgeschwindigkeiten der Gitterstrukturabschnitte aus.

Bei den Ausführungsbeispielen gemäß Figuren 1 bis 3 ist ferner zu erkennen, dass im Bereich des ersten axialen Endes 13a der Gitterstruktur 10 ein trichterförmiger Abschnitt 15 gebildet ist. Der trichterförmige Abschnitt 15 ergibt sich insbesondere aus der Zusammenführung der Drahtelemente 11 zur ersten Verbindungsstelle 12a. Der trichterförmige Abschnitt 15 ermöglicht auf einfache Weise das Zurückführen der Gitterstruktur 10 in ein Zufuhrsystem. Dabei dient der trichterförmige Abschnitt 15 im Wesentlichen als Zentrierung und Einführhilfe. Der trichterförmige Abschnitt 15 kann zumindest teilweise mit einer fluiddichten Abdeckung versehen sein. Ferner kann vorgesehen sein, dass die Gitterstruktur 10 mit einer Absaugvorrichtung gekoppelt ist. Durch die Absaugvorrichtung kann innerhalb der Gitterstruktur 10 ein Unterdruck erzeugt werden, der abgelöste Konkrementpartikel aus dem Körperhohlraum zieht. Durch eine fluiddichte Abdeckung zumindest im Bereich des trichterförmigen Abschnitts 15 wird erreicht, dass der Unterdruck vorwiegend auf die Gefäßwände im Bereich der Gitterstruktur 10 wirkt. Der Einfluss der Absaugvorrichtung kann auf diese Weise lokal begrenzt werden. Beim Einsatz der Vorrichtung in Blutgefäßen kann beispielsweise vermieden werden, dass Blut aus weiteren Gefäßbereichen außerhalb des Behandlungsortes abgesaugt wird.

Die hier beschriebene medizinische Vorrichtung ist vorteilhaft zur endovaskulären Behandlung einsetzbar. Dabei kann die Rotation bzw. Torsion der Gitterstruktur 10 bzw. der Gitterstrukturabschnitte besonders vorteilhaft zum Lösen von Konkrementen, insbesondere Thromben oder Plaque, genutzt werden. Besonders vorteilhaft kann die Rotation durch eine axiale Schiebe- bzw. Zugbewegung des Anwenders ausgelöst werden. Der Behandlungsvorgang, insbesondere die Abschabung von Partikeln durch die Rotation der Gitterstruktur 10, ist somit für den Anwender einfach kontrollierbar und steuerbar.

Die medizinische Vorrichtung wird vorzugsweise mittels eines Zufuhrsystems an den Behandlungsort geführt. Das Zufuhrsystem kann eine Schleuse bzw. allgemein einen Schlauch umfassen, der die Gitterstruktur 10 im komprimierten Zustand fixiert. Die Schleuse bzw. der Schlauch kann an einen Katheter angeschlossen bzw. angedockt werden. Alternativ kann die Gitterstruktur 10 direkt innerhalb eines Katheters angeordnet sein. Zur Expansion der Gitterstruktur 10 bestehen im Wesentlichen zwei Möglichkeiten. Einerseits kann die Gitterstruktur 10 axial in distaler Richtung bezüglich des Katheters geschoben werden, wogegen der Katheter ortsfest gehalten wird. Die Gitterstruktur 10 wird somit aus der Katheterspitze geschoben. Andererseits kann die Gitterstruktur 10 ortsfest gehalten werden und der Katheter in proximaler Richtung gezogen werden, um die Gitterstruktur 10 freizugeben.

Um das Abtrennen der Konkremente zu erleichtern, können Teile der Gitterstruktur 10 Schneidbereiche, insbesondere Schneidkanten bzw. Schneidflächen aufweisen. Beispielsweise können die Filamente 11a, 11b Schneidkanten umfassen, so dass bei Torsion bzw. Rotation der Gitterstruktur bzw. der Gitterstrukturabschnitte eine erleichterte Abschabung von Konkrementpartikeln erfolgt. Ferner kann das zweite axiale Ende 13b der Gitterstruktur 10 Schneidspitzen aufweisen. Die Schneidspitzen können beispielsweise durch Schneidkanten im Bereich der Verbindungsstelle 12a, 12b am zweiten axialen Ende 13b ausgebildet sein. Alternativ kann das zweite axiale Ende 13b der Gitterstruktur 10 abgerundete Kanten umfassen, so dass eine im Wesentlichen atraumatische Geflechtspitze gebildet ist.

Vorzugsweise werden Konkremente bei der Behandlung schichtweise abgetragen. Dazu kann es vorteilhaft sein, die Gitterstruktur 10 vergleichsweise grobmaschig auszubilden. Eine derartige Struktur eignet sich insbesondere zur Abtragung von Plaque. Für das Entfernen von Plaque ist ferner eine schneidende Gitterstruktur 10 vorteilhaft. Die Entlassung der Gitterstruktur 10 aus einem Zufuhrsystem erfolgt bei der Behandlung von Plaque vorteilhafterweise durch Zurückziehen des Zufuhrsystems bzw. Katheters ohne Veränderung der Ortslage der Gitterstruktur 10.

Die medizinische Vorrichtung eignet sich auch zur Zertrümmerung von Thromben. Im Unterschied zur Behandlung von Plaque, bei der eine vollständige Expansion der Gitterstruktur 10 vorteilhaft ist, reicht bei der Thrombenbehandlung eine Teilexpansion der Gitterstruktur 10 aus. Die Rotation der Gitterstruktur 10 kann bei der Zertrümmerung von Thromben beispielsweise derart genutzt werden, dass sich das zweite axiale Ende 13b der Gitterstruktur 10 rotierend in den Thrombus einschneidet. Die Vorrichtung kann also im Wesentlichen nach Art eines Bohrers eingesetzt werden. In Kombination mit einer Absaugvorrichtung können während der Behandlung abgelöste Partikel entfernt bzw. abgesaugt werden. Vorteilhaft kann die Gitterstruktur 10 zu diesem Zweck eine fluiddichte Abdeckung aufweisen, so dass die Saugwirkung auf den Behandlungsbereich begrenzt ist oder zumindest vermieden wird, dass weitere Gefäßbereiche durch die Absaugung beeinträchtigt werden.

Die Zufuhr der medizinischen Vorrichtung zu einem medizinischen Behandlungsort erfolgt besonders vorteilhaft durch einen Katheter. Bevorzugt sind Katheter, die einen Innendurchmesser aufweisen, der kleiner als das 0,5-fache, insbesondere kleiner als das 0,3-fache, insbesondere kleiner als das 0,2-fache, insbesondere kleiner als das 0,1-fache, insbesondere kleiner als das 0,05-fache des Ruhedurchmessers der Gitterstruktur 10 beträgt. Der Ruhedurchmesser entspricht dem Querschnittsdurchmesser, den die Gitterstruktur 10 ohne Einwirkung äußerer Kräfte selbsttätig einnimmt.

Das Verhältnis der Rotation zur translatorischen Bewegung bzw. Längenänderung der Gitterstruktur 10 bzw. von Abschnitten der Gitterstruktur 10 wird als Rotationsrate bezeichnet. Vorzugsweise beträgt die Rotationsrate mindestens 2%/mm, insbesondere wenigstens 5%/mm, insbesondere 10%/mm, insbesondere wenigstens 20%/mm, insbesondere wenigstens 30%/mm, insbesondere wenigstens 40%/mm, insbesondere wenigstens 50%/mm. Dabei entspricht eine Rotation von 100% einer Drehung eines Punktes, beispielsweise einer Verbindungsstelle 12a, 12b, der Gitterstruktur 10 um 360° um die gemeinsame Rotationsachse L.

Im komprimierten Zustand weist die Gitterstruktur 10 im Wesentlichen gestreckte Filamente 11a, 11b auf. Das bedeutet, dass im komprimierten Zustand der Abstand A der Verbindungsstellen 12a, 12b derart vergrößert ist, das die Filamente 11a, 11b im Wesentlichen benachbart bzw. quasiparallel zueinander angeordnet sind. Bei der Expansion bewirken die inneren Kräfte der Gitterstruktur 10, dass die Filamente 11a, 11b in die jeweilige Spiralform zurückgeführt werden, wobei sich der Abstand A zwischen den Verbindungsstellen 12a, 12b verkürzt. Gleichzeitig rotiert die zweite Verbindungsstelle 12b gegenüber der ersten Verbindungsstelle 12a. Vorzugsweise rotiert die zweite Verbindungsstelle 12b gegenüber der ersten Verbindungsstelle 12a wenigstens einmal, insbesondere wenigstens zweimal, insbesondere wenigstens dreimal, insbesondere wenigstens fünfmal, insbesondere wenigstens zehnmal um die gemeinsame Rotationsachse L.

Der erste Flechtwinkel α beträgt vorzugsweise zwischen 30° und 60°, insbesondere zwischen 40° und 50°, besonders bevorzugte 45°. Der zweite Flechtwinkel β kann größer als der erste Flechtwinkel α sein, wodurch die Vorrichtung eine erhöhte Flexibilität und Feinmaschigkeit aufweist. Durch die Feinmaschigkeit werden Expansionskräfte schonend verteilt und eine verbesserte Filterfunktion bereitgestellt. Der zweite Flechtwinkel β kann kleiner als der erste Flechtwinkel α sein, wodurch die Verkürzung der Gitterstruktur 10 bei der Expansion (Foreshortening) reduziert wird. Die Handhabung der Vorrichtung wird somit verbessert. Durch den kleineren zweiten Flechtwinkel β wird eine relativ grobmaschige Struktur gebildet, die einen verbesserten Schneideffekt aufweist. Das Fangen und/oder Einsaugen von Partikeln wird somit vorteilhaft unterstützt.

Der Winkelunterschied zwischen dem ersten Flechtwinkel a, und dem zweiten Flechtwinkel β beträgt vorzugsweise mehr als 10°, insbesondere mehr als 20°, insbesondere mehr als 30°, insbesondere mehr als 40°, insbesondere mehr als 50°. Bei Einsatz der Vorrichtung in kleineren Körperhohlräumen, beispielsweise in zerebralen Gefäßen, ist eine kleinere Rotationsrate gewünscht. Dazu ist ein Winkelunterschied zwischen dem ersten Flechtwinkel α und dem zweiten Flechtwinkel β vorteilhaft, der weniger als 20°, insbesondere weniger als 15°, insbesondere weniger als 10°, insbesondere weniger als 8°, insbesondere weniger als 5°, insbesondere weniger als 4°, insbesondere weniger als 3°, insbesondere weniger als 2°, beträgt.

Die Längsabschnitte 14a, 14b weisen vorzugsweise eine Länge von höchstens 20 mm, insbesondere höchstens 10 mm, insbesondere höchstens 8 mm, insbesondere höchstens 5 mm, insbesondere höchstens 4 mm, insbesondere höchstens 3 mm, insbesondere höchstens 2 mm, auf. Durch mehrere, vergleichsweise kurze Längsabschnitte 14a, 14b wird eine häufige Änderung der Rotationsrate bzw. Rotationsrichtung bei der Expansion der Gitterstruktur 10 erreicht. Vorzugsweise sind wenigstens zwei, insbesondere wenigstens drei, insbesondere wenigstens fünf, insbesondere wenigstens sieben, insbesondere wenigstens zehn Längsabschnitte 14a, 14b mit unterschiedlicher Rotationsrate und/oder unterschiedlicher Rotationsrichtung bezogen auf die jeweils benachbarten Längsabschnitte vorgesehen. Die Rotations- bzw. Torsionsrichtung und/oder die Rotations- bzw. Torsionsrate variiert wenigstens einmal, insbesondere wenigstens zweimal, insbesondere wenigstens viermal, insbesondere wenigstens sechsmal, insbesondere wenigstens neunmal, entlang der Gitterstruktur 10.

Es ist möglich, dass sich die Rotationsrate entlang der Gitterstruktur 10 ändert, insbesondere kontinuierlich ändert. In einem ersten Längsabschnitt 14a kann die Rotationsrate beispielsweise vergleichsweise groß sein. Die Kontaktfläche mit dem Konkrement kann dabei vergleichsweise klein sein, so dass die Reibungskräfte zwischen dem ersten Längsabschnitt 14a und dem Konkrement reduziert sind. In nachfolgenden Längsabschnitten 14a, 14b kann die Rotationsrate reduziert sein. Bei der weiteren Entlassung der Gitterstruktur 10 gelangt eine größere Fläche der Gitterstruktur 10 mit dem Konkrement in Anlage, wodurch die Reibungskräfte erhöht werden. Bei höherer Reibung kann eine reduzierte Rotationsrate vorteilhaft sein.

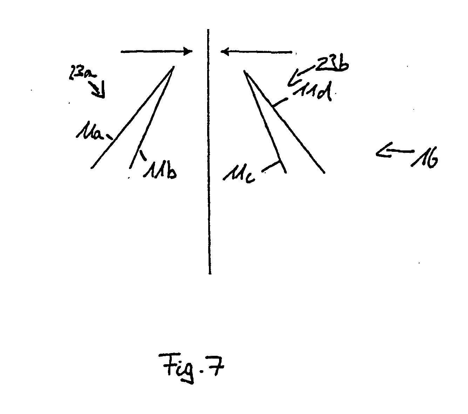

Ein weiteres Ausführungsbeispiel, bei dem der Rotationseffekt zu Betätigung eines Funktionselements, bspw. eines Greifabschnitts 22 benutzt wird, ist in Fig. 7 dargestellt. Den Antrieb des Funktionselements bilden zwei konzentrisch ineinander angeordnete hohlzylindrische Gitterstrukturen, die jeweils erfindungsgemäß ausgebildet sind und somit durch eine Durchmesseränderung tordierbar sind. Alle in der Anmeldung offenbarten Merkmale werden auch im Zusammenhang mit den beiden Gitterstrukturen offenbart. Konkret sind die beiden Gitterstrukturen so ausgebildet, dass beide Gitterstrukturen bei einer Durchmesseränderung gegensinnig rotieren.

Die beiden Gitterstrukturen sind jeweils mit einer Komponente des Funktionselements verbunden, so dass die beiden Komponente gegensinnig bewegbar sind. Konkret bilden die beiden Komponenten zwei Zangenabschnitte 23a, 23b. Ein erster Zangenabschnitt 23a ist durch zwei erste Filamente 11a, 11b und ein zweiter Zangenabschnitt 23b durch zwei zweite Filamente 11c, 11d gebildet, die in Längsrichtung der Vorrichtung spitz zusammenlaufen und am axialen Ende der Zangenabschnitte 23a, 23b jeweils verbunden oder umgelenkt sind. Die ersten Filamente 11a, 11b sind mit der ersten Gitterstruktur und die zweiten Filamente 11c, 11d mit der zweiten Gitterstruktur verbunden. Dazu sind die Filamente der Gitterstrukturen verlängert. Andere Verbindungen, bspw. durch Schweißen, Kleben, Crimpen usw. sind möglich. Eine Rotationsbewegung der ersten Gitterstruktur führt zu einer Bewegung des ersten Zangenabschnitts 23a. Eine Rotationsbewegung der zweiten Gitterstruktur führt zu einer Bewegung des zweiten Zangenabschnitts 23a. Die beiden Zangenabschnitte 23a, 23b bewegen sich dabei aufeinander zu (Fig. 7) oder voneinander weg auf Grund der gegensinnigen Rotation der jeweils zugehörigen Gitterstrukturen.

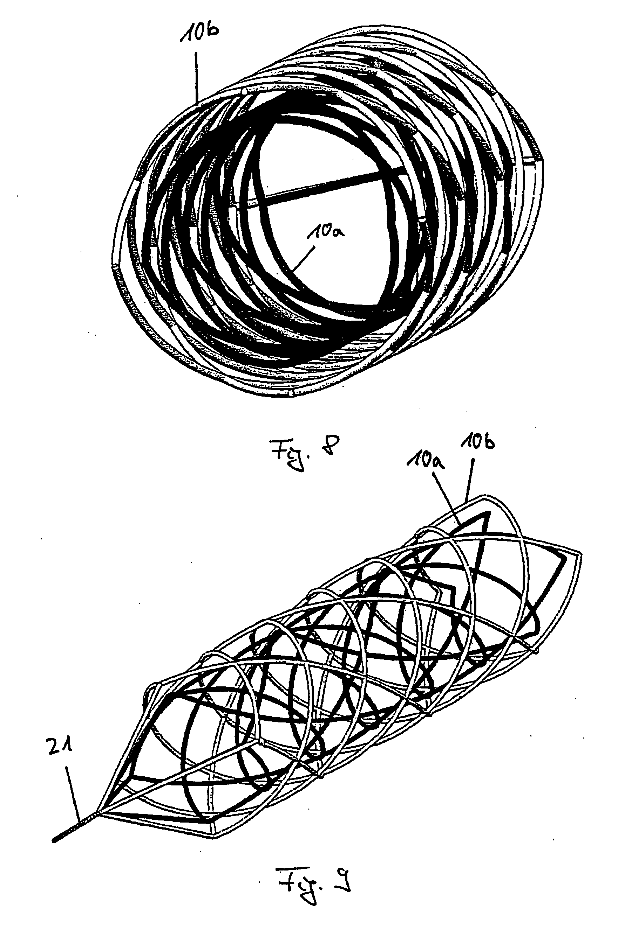

Figuren 8 und 9 zeigen ein weiteres Ausführungsbeispiel bei dem zwei relativ zueinander tordierbare Gitterstrukturen 10a, 10b konzentrisch ineinander angeordnet sind. Mehr als zwei, insbesondere drei konzentrisch ineinander angeordnete Gitterstrukturen sind möglich. Die hohlzylindrischen Gitterstrukturen 10a, 10b bilden eine stent-in stent Anordnung.

Die Relativbewegung zwischen den Gitterstrukturen 10a, 10b kann durch eine Drehung der äußeren Gitterstruktur 10b bei stillstehender innerer Gitterstruktur 10a oder durch eine Drehung der inneren Gitterstruktur 10a bei stillstehender äußerer Gitterstruktur 10b oder durch Drehung beider Gitterstrukturen 10a, 10b erfolgen. Im letzteren Fall können die Gitterstrukturen 10a, 10b gegensinnig oder gleichsinnig mit unterschiedlichen Geschwindigkeiten tordieren.

Für die Drehbewegung sind die innere Gitterstruktur 10a und/oder die äußere Gitterstruktur 10b erfindungsgemäß, insbesondere nach einem der in den Unteransprüchen angegebenen Ausführungsformen bzw. nach einem der in der Beschreibung genannten Beispiele ausgeführt.

Wie gut in Fig. 9 zu erkennen, sind beide Gitterstrukturen 10a, 10b mit demselben Zuführelement, insbesondere mit demselben Führungsdraht 21 verbunden. Dadurch wird die Handhabung der Mehrfachstruktur vereinfacht, weil beide Gitterstrukturen 10a, 10b zusammen in axialer Richtung bewegt und die Relativrotation zwischen den Gitterstrukturen 10a, 10b durch die gemeinsame axiale Bewegung bewirkt wird.

Die beiden Gitterstrukturen 10a, 10b können im expandierten Zustand in Kontakt miteinander sein, also aneinander anliegen. Wie in Fig. 8 dargestellt, kann aber auch ein Spalt, insbesondere ein Ringspalt im Ruhezustand, d.h. im expandierten Zustand ohne Einwirkung äußerer Kräfte und/oder im expandierten Zustand im Gefäß zwischen den Gitterstrukturen 10a, 10b ausgebildet sein. Durch den Spalt kann die Größe der getrennten Partikel bzw. Thrombenteile oder die Größe der Thrombenbereiche, die sich verfangen sollen, bestimmt werden. Vorzugsweise hat der Spalt zwischen den Gitterstrukturen 10a, 10b eine Breite von mindestens 0,1 mm, insbesondere eine Breite von mindestens 0,2 mm, eine Breite von mindestens 0,3 mm, eine Breite von mindestens 0,5 mm, eine Breite von mindestens 0,7 mm, eine Breite von mindestens 1 mm. Die äußere Gitterstruktur 10a hat z.B. einen Durchmesser von 3,5 mm. Die innere Gitterstruktur 10b hat z.B. einen Durchmesser von 2,5 mm. Es ergibt sich ein Spalt von ca. 0,5 mm, wobei auch die Stärke der Wandung eine Rolle spielt.

Die beiden Gitterstrukturen 10a, 10b können in einem Bereich miteinander verbunden sein, bspw. in einem distalen Bereich oder in einem mittleren Bereich der jeweiligen Gitterstruktur 10a, 10b. Dies bedeutet, dass die Gitterstrukturen einerseits proximal aufgrund des gemeinsamen Führungsdrahtes 21 und andererseits in einem anderen vom proximalen Ende beabstandeten Bereich, also an zwei Stellen, miteinander verbunden sind. Dies ist zum Beispiel möglich, wenn die Tordierung der beiden Gitterstrukturen 10a, 10b so eingestellt ist, dass die beiden Verbindungsbereiche, bzw. Verbindungsstellen nicht relativ zueinander rotieren. Dies bedeutet, dass die Gesamtstruktur entlang der Längsachse so rotiert, dass die Gesamtrotation null ist und die Verbindungsbereiche ihre Relativlage nicht ändern, so dass das Geflecht nicht verzerrt wird. Hierzu wird auf die Erläuterungen im Zusammenhang mit den Fig. 2 und 3 verwiesen. Konkret dreht sich z.B. im proximalen Bereich am Führungsdraht eine der beiden Gitterstrukturen 10a, 10b gegen den Uhrzeigersinn und die andere der beiden Gitterstrukturen 10a, 10b im Uhrzeigersinn. Im distalen Bereich erfolgt die Relativdrehung entsprechend anders, so dass sich die Relativrotation insgesamt aufhebt.

Das Ausführungsbeispiel gemäß Fig. 7 kann mit dem Ausführungsbeispiel gemäß Fig. 8, 9 kombiniert werden, wobei einer der beiden Zangenabschnitte 23a, 23b mit der inneren Gitterstruktur 10a und der andere der beiden Zangenabschnitte 23a, 23b mit der äußeren Gitterstruktur 10b verbunden ist. Es ist möglich, dass die Zangenabschnitte 23a, 23b separate Bauteile sind , die mit der jeweiligen Gitterstruktur 10a, 10b verbunden sind. Es ist auch möglich, dass die Zangenabschnitte 23a, 23b selbst zur jeweiligen Gitterstruktur 10a, 10b gehören, bzw. Teil der Gitterstruktur 10a, 10b sind.

Die Ausführungsbeispiele gemäß Fig. 8, 9 umfassen geflochtene Gitterstrukturen. Es ist auch möglich, Gitterstrukturen mit Stegen anstelle der geflochtenen Gitterstrukturen zu verwenden. Die Stege bzw. die geflochtenen Filamente bilden jeweils die Schneidelemente. Der Stegwinkel ersetzt den Flechtwinkel. Die im Zusammenhang mit der geflochtenen Struktur offenbarten Merkmale werden im Zusammenhang mit der stegförmigen Gitterstruktur offenbart. Die stegförmigen Gitterstrukturen können in an sich bekannter Weise, bspw. durch Schneiden, insbesondere Laserschneiden, Ätzen oder durch PVD-Verfahren, insbesondere durch Sputtern, hergestellt werden.

Allgemein gilt, dass alle vorstehend beschriebenen Gitterstrukturen 10, 10a, 10b durch Verflechten von Drahtelementen bzw. Drähten hergestellt werden können. Um eine vergleichsweise dünne Wandstärke bereitzustellen, sind Drähte mit einem Durchmesser von höchstens 200 µm, insbesondere höchstens 150 µm, insbesondere höchstens 100 µm, insbesondere höchstens 75 µm, insbesondere höchstens 50 µm, bevorzugt. Als Drahtmateriafien kommen Nickel-Titan-Legierungen, Chrom-Kobalt-Legierungen, wie beispielsweise Phynox, Elgiloy, nichtrostende Stähle (z.B. 316 LVM), Platin und/oder Platinlegierungen, insbesondere Platin-Iridium-Legierungen, Magnesium und/oder Magnesiumlegierungen sowie Wolfram bzw. Wolfram-Legierungen, insbesondere Titan-Wolfram-Legierungen oder Wolfram-Rhenium-Legierungen in Frage. Generell können auch Werkstoffe wie beispielsweise Magnesium, Eisen oder Wolfram und deren Legierungen verwendet werden, die bioadsorbierbare Eigenschaften aufweisen. Es ist auch möglich, zur Bildung der Gitterstruktur 10 Kunststofffäden, wie beispielsweise Dynema, zu verwenden. Die Gitterstruktur 10 kann auch aus bioresorbierbaren Kunststoffen hergestellt sein.

Die Herstellung der Gitterstruktur 10 erfolgt vorteilhafterweise auf einer Flechtmaschine. Die Flechtmaschine wird mit Drahtelementen 11 bestückt, die zur Bildung der zweiten Verbindungsstelle 12b umgelenkt werden. Die beiden sich von der zweiten Verbindungsstelle 12b erstreckenden Drahtabschnitte bilden einerseits das erste Filament 11a und andererseits das zweite Filament 11b. Die Filamente 11a, 11b werden durch unterschiedliche Rotation von Klöppeln der Flechtmaschine derart in eine Spiralform überführt, dass sich unterschiedliche Flechtwinkel a, β einstellen. Die Rotation der Klöppel kann während des Flechtvorgangs variieren, so dass sich eine kontinuierliche Änderung der Flechtwinkel α, β einstellt. Zur Führung der Filamente 11a, 11b kann ein Flechtdorn vorgesehen sein, um den die Filamente 11a, 11b spiralförmig gewickelt werden.

Zur Herstellung einer Gitterstruktur 10 mit einer schrittweisen oder diskreten Flechtwinkeländerung weist der Flechtdorn vorzugsweise Stifte auf, die sich radial von dem Flechtdorn erstrecken. Die Filamente 11a, 11b werden an den Stiften umgelenkt, so dass sich der Flechtwinkel α, β ändert. Es ist auch möglich, die Stifte zur Bildung einer kontinuierlichen oder quasi-kontinuierlichen Flechtwinkeländerung zu nutzen. Dazu sind vorzugsweise mehrere Stifte in eng benachbart zueinander angeordnet. Anstelle der Stifte kann der Flechtdorn auch eine Negativform für die herzustellende Gitterstruktur 10 aufweisen. Die Negativform kann gitterartig angeordnete Nuten oder Rippen umfassen in die bzw. entlang derer die Filamente 11a, 11b gewickelt werden. Die Nuten oder Rippen können einen kontinuierlich oder diskret variierenden Neigungswinkel bezüglich der Rotationsachse des Flechtdorns aufweisen. Die Filamente 11a, 11b werden auf diese Weise mit unterschiedlichen Flechtwinkein α, β versehen. Vorzugsweise werden die Filamente 11a, 11b vor dem Entfernen des Flechtdorns einer Wärmebehandlung unterzogen, so dass die gewickelte Form aufrechterhalten wird.

Es ist auch möglich, anstelle der geflochtenen Gitterstrukturen stegförmig aufgebaute Gitterstrukturen 10a, 10b zu verwenden, die bspw. durch Schneiden, insbesondere Laserschneiden hergestellt sind. Dies gilt für alle Ausführungsbeispiele.

Im Zusammenhang mit der Erfindung und mit allen Ausführungsbeispielen wird offenbart, dass das Haltelement mit der Gitterstruktur konkret derart verbunden ist, dass die Filamente bzw. Elemente insbesondere am proximalen Ende der Gitterstruktur, in das Halteelement münden bzw. im Haltelement zusammengeführt sind, so dass zumindest im Halteelement keine oder zumindest keine signifikante Relativbewegung zwischen den Filamenten bzw. Element stattfindet, wenn die Gitterstruktur verformt wird. Die Filamente bzw. Elemente sind im Bereich des Halteelements relativ zueinander festgelegt. Außerhalb des Haltelements ist die Relativbewegung möglich. Die Filamente bzw. Elemente können im Haltelement bspw. dadurch zusarrimengeführt sein, dass diese verflochten verdrillt oder anderweitig miteinander verbinden sind. Dabei bildet die Verbindung der Filamente bzw. Elemente untereinander das Halteelement. Alternativ oder zusätzlich kann das Haltelement ein separates Bauteil, wie eine Hülse sein, die die Filamente bzw. Elemente fest hält, bspw. durch Crimpen. Zusätzlich oder alternativ können die Filamenten bzw. Elemente stoffschlüssig miteinander verbunden sein. Die vorstehenden Befestigungen können miteinander kombiniert sein.

Die Filamente bzw. Elemente sind generell im Bereich des Halteelements untereinander fixiert. Die Ortslage der Enden der Filamente bzw. Elemente im Bereich des Haltelements und/oder die Ortslage der Abschnitte der Filamente bzw. Elemente im Bereich des Haltelements relativ zueinander ist fixiert, wenn sich die Gitterstruktur verformt.

### Bezugszeichenliste

- 10: Gitterstruktur
- 10a: inneren Gitterstruktur
- 10b: äußere Gitterstruktur
- 11: Drahtelement
- 11a: erstes Filament
- 11b: zweites Filament
- 12a: erste Verbindungsstelle
- 12b: zweite Verbindungsstelle
- 13a: erstes axiales Ende
- 13b: zweites axiales Ende
- 14a: erster Längsabschnitt
- 14b: zweiter Längsabschnitt
- 15: trichterförmiger Abschnitt
- 16: Kreuzungspunkt
- 17: Masche
- 18: Übergangsstelle
- 20: Halteelement
- 21: Führungsdraht
- 22: Greifabschnitt
- α: erster Flechtwinkel
- β: zweiter Flechtwinkel
- L: Rotationsachse
- A: Abstand
- πD: Umfang
- x: Umfangsrichtung
- y: Axialrichtung

## Patentansprüche

1. Medizinische Vorrichtung zum Lösen von Konkrementen in Körperhohlorganen mit wenigstens einer rotationssymmetrischen Gitterstruktur (10), die mit einem Halteelement (20) drehfest verbunden ist und wenigstens zwei um eine gemeinsame Rotationsachse L gewundene und sich kreuzende Filamente (11a, 11b) aufweist, die an wenigstens zwei in Längsrichtung der Gitterstruktur (10) beabstandet angeordneten Verbindungsstellen (12a, 12b) miteinander verbunden sind, wobei ein erstes Filament (11a) einen ersten Flechtwinkel a und ein zweites Filament (11b) einen zweiten Flechtwinkel β bildet, der zumindest abschnittsweise von dem ersten Flechtwinkel α verschieden ist derart, dass eine erste Verbindungsstelle (12a) beim Übergang der Gitterstruktur (10) von einem radial komprimierten in einen radial expandierten Zustand, oder umgekehrt, gegenüber einer zweiten Verbindungsstelle (12b) um die gemeinsame Rotationsachse L verdreht wird.

2. Medizinische Vorrichtung nach Anspruch 1,
**dadurch gekennzeichnet, dass**
das Halteelement (20) ein flexibles Führungsmittel, insbesondere einen Führungsdraht (21), aufweist.

3. Medizinische Vorrichtung nach Anspruch 1 oder 2,
**dadurch gekennzeichnet, dass**
die Gitterstruktur (10) ein erstes axiales Ende (13a) und ein zweites axiales Ende (13b) aufweist, wobei das erste axiale Ende (13a) mit dem Halteelement (20) drehfest verbunden und das zweite axiale Ende (13b) frei angeordnet ist.

4. Medizinische Vorrichtung nach Anspruch 3,
**dadurch gekennzeichnet, dass**
die erste Verbindungsstelle (12a) am zweiten axialen Ende (13b) und die zweite Verbindungsstelle (12b) am ersten axialen Ende (13a) angeordnet ist.

5. Medizinische Vorrichtung nach wenigstens einem der Ansprüche 1 bis 4,
**dadurch gekennzeichnet,dass**
das erste Filament (11a) mit dem zweiten Filament (11b) verflochten ist.

6. Medizinische Vorrichtung nach wenigstens einem der Ansprüche 1 bis 5,
**dadurch gekennzeichnet, dass**
die Gitterstruktur (10) mehrere erste Filamente (11a) und/oder mehrere zweite Filamente (11b) umfasst, wobei zumindest abschnittsweise die ersten Filamente (11a) jeweils den gleichen ersten Flechtwinkel α und die zweiten Filamente (11b) jeweils den gleichen zweiten Flechtwinkel β aufweisen.

7. Medizinische Vorrichtung nach wenigstens einem der Ansprüche 1 bis 6,
**dadurch gekennzeichnet, dass**
der erste Flechtwinkel α und/oder der zweite Flechtwinkel β zumindest abschnittsweise entlang der Gitterstruktur (10) variiert.

8. Medizinische Vorrichtung nach wenigstens einem der Ansprüche 1 bis 7,
**dadurch gekennzeichnet, dass**
die Gitterstruktur (10) wenigstens einen ersten Längsabschnitt (14a) und wenigstens einen zweiten Längsabschnitt (14b) aufweist, wobei die Flechtwinkel α, β im ersten Längsabschnitt (14a) und im zweiten Längsabschnitt (14b) eingestellt sind derart, dass der erste Längsabschnitt (14a) beim Übergang der Gitterstruktur (10) vom radial komprimierten zum radial expandierten Zustand, oder umgekehrt, gegensinnig zum zweiten Längsabschnitt (14b) um die gemeinsame Rotationsachse L verdreht wird.

9. Medizinische Vorrichtung nach Anspruch 8,
**dadurch gekennzeichnet, dass**
das erste Filament (11a) und das zweite Filament (11b) im ersten Längsabschnitt (14a) und im zweiten Längsabschnitt (14b) jeweils unterschiedliche Abschnittslängen und über die gesamte Länge der Gitterstruktur (10) im Wesentlichen die gleiche Gesamtlänge aufweisen.

10. Medizinische Vorrichtung nach Anspruch 8 oder 9,
**dadurch gekennzeichnet, dass**
der erste Flechtwinkel α im ersten Längsabschnitt (14a) größer und im zweiten Längsabschnitt (14b) kleiner als der zweite Flechtwinkel β ist.

11. Medizinische Vorrichtung nach wenigstens einem der Ansprüche 1 bis 10,
**dadurch gekennzeichnet, dass**
die Filamente (11a, 11b) zumindest abschnittsweise wenigstens einen Schneidebereich, insbesondere eine Schneidefläche oder eine Schneidekante, aufweisen.

12. Medizinische Vorrichtung nach wenigstens einem der Ansprüche 1 bis 11,
**dadurch gekennzeichnet, dass**
die Gitterstruktur (10) im Bereich des ersten axialen Endes einen trichterförmigen Abschnitt (15) aufweist.

13. Medizinische Vorrichtung nach wenigstens einem der Ansprüche 1 bis 12,
**dadurch gekennzeichnet, dass**
die Gitterstruktur (10), insbesondere der trichterförmige Abschnitt (15), wenigstens abschnittsweise eine fluiddichte Abdeckung aufweist.

14. Medizinische Vorrichtung mit einer ersten Gitterstruktur (10b) gemäß einem der vorhergehenden Ansprüche und wenigstens einer zweiten Gitterstruktur (10a) gemäß einem der vorhergehenden Ansprüche, die koaxial innerhalb der ersten Gitterstruktur (10b) angeordnet ist, wobei die Filamente (11a, 11b) der ersten Gitterstruktur (10b) gegenüber den Filamenten (11c, 11d) der zweiten Gitterstruktur (10a) unterschiedliche Flechtwinkel α, β, α', β' aufweisen derart, dass beim Übergang der Gitterstrukturen (10a, 10b) vom radial komprimierten zum radial expandierten Zustand, oder umgekehrt, die erste Gitterstruktur (10b) und die zweite Gitterstruktur (10a) relativ zueinander verdrehbar sind.

15. Medizinische Vorrichtung nach Anspruch 14,
**dadurch gekennzeichnet, dass**
die ersten Gitterstruktur (10b) und die zweite Gitterstruktur (10a) jeweils wenigstens zwei gleichsinnig um die gemeinsame Rotationsachse gewundene Filamente (11a, 11b, 11c, 11d) aufweisen, wobei die Filamente (11a, 11b) der ersten Gitterstruktur (10b) zumindest am gemeinsamen zweiten axialen Ende (13b) der Gitterstrukturen (10a, 10b) gegenüber den Filamenten (11c, 11d) der zweiten Gitterstruktur (10a) gegensinnig um die gemeinsame Rotationsachse gewunden sind, um einen scherenartig bzw. zangenartig betätigbaren Greifabschnitt (22) zu bilden.

16. Verfahren zum Herstellen einer medizinischen Vorrichtung nach Anspruch 1 umfassend die folgenden Schritte:
- Bereitstellen einer Flechtmaschine, die um eine gemeinsame Rotationsachse L rotierende Klöppel aufweist;
- Verbinden des ersten Filaments (11a) mit einem ersten Klöppel und des zweiten Filaments (11b) mit einem zweiten Klöppel;
- Einstellen der Rotation der Klöppel derart, dass das erste Filament (11a) mit dem ersten Flechtwinkel α und das zweite Filament (11b) mit dem zweiten Flechtwinkel β zur Bildung der rotationssymmetrischen Gitterstruktur (10) gegensinnig um die gemeinsame Rotationsachse L gewickelt werden;
- Verbinden des ersten Filaments (11a) mit dem zweiten Filament (11b) an der ersten Verbindungsstelle (12a) und an der von der ersten Verbindungsstelle (12a) beabstandet angeordneten zweiten Verbindungsstelle (12b)
oder
Umlenken eines gemeinsamen Drahtelements (11), das das erste Filament (11a) und das zweite Filament (11b) bildet, zur Bildung der ersten Verbindungsstelle (12a) und Verbinden von jeweils einem freien Ende des ersten Filaments (11a) mit einem freien Ende des zweiten Filaments (11b) zur Bildung der von der ersten Verbindungsstelle (12a) beabstandet angeordneten zweiten Verbindungsstelle (12b);
- Verbinden eines ersten axialen Endes (13a), insbesondere eines proximalen Endes, der Gitterstruktur (10) mit dem Halteelement (20).

17. Medizinische Vorrichtung zum Lösen von Konkrementen in Körperhohlorganen mit wenigstens einer rotationssymmetrischen Gitterstruktur (10), die mit einem Halteelement (20) drehfest verbunden ist und wenigstens zwei um eine gemeinsame Rotationsachse L spiralförmig angeordnete und sich kreuzende Gitterstege (11a, 11b) aufweist, die an wenigstens zwei in Längsrichtung der Gitterstruktur (10) beabstandet angeordneten Verbindungsstellen (12a, 12b) miteinander verbunden sind, wobei ein erster Gittersteg (11a) einen ersten Stegwinkel α und ein zweiter Gittersteg (11b) einen zweiten Stegwinkel β bildet, der zumindest abschnittsweise von dem ersten Stegwinkel α verschieden ist derart, dass eine erste Verbindungsstelle (12a) beim Übergang der Gitterstruktur (10) von einem radial komprimierten in einen radial expandierten Zustand, oder umgekehrt, gegenüber einer zweiten Verbindungsstelle (12b) um die gemeinsame Rotationsachse L verdreht wird.

18. Behandlungssystem mit einer medizinischen Vorrichtung gemäß einem der vorhergehenden Ansprüche 1 bis 15 und 17 und einem Katheter, in dem das Halteelement (20) längsverschiebbar angeordnet ist, wobei das Halteelement (20) mit einem ersten axialen Ende (13a) der Gitterstruktur (10) verbunden ist, das einem proximalen Ende des Katheters zugewandt ist.

19. Verfahren zum Herstellen eines Behandlungssystems, gemäß Anspruch 18, umfassend die folgenden Schritte:
- Bereitstellen einer Schleuse mit einem proximalen Ende und einem distalen Ende;
- Anordnen des Halteelements (20) in der Schleuse, wobei das Halteelement (20) vom distalen Ende zum proximalen Ende der Schleuse geführt wird;
- Überführen der Gitterstruktur (10) von einem radial expandierten in einen radial komprimierten Zustand durch Einziehen der Gitterstruktur (10) in das distale Ende der Schleuse, wobei die zweite, insbesondere an einem zweiten, freien axialen Ende (13b) der Gitterstruktur (10) angeordnete, Verbindungsstelle (12b) der Gitterstege, insbesondere der Filamente (11a, 11b) relativ zum Halteelement (20) um die gemeinsame Rotationsachse L verdreht wird.

## Claims

1. Medical device for detaching concretions in hollow organs of the body, having at least one rotationally symmetrical lattice structure (10) which is joined to a holding element (20) for conjoint rotation therewith and which has at least two filaments (11a, 11 b) which cross one another and are wound around a common axis of rotation L and which are joined to one another at at least two joining locations (12a, 12b) arranged spaced apart in the longitudinal direction of the lattice structure (10), a first filament (11 a) forming a first twist angle α and a second filament (11b) forming a second twist angle β which, at least regionally, differs from the first twist angle α in such a way that, on changing of the lattice structure (10) from a radially compressed to a radially expanded state, or vice-versa, a first joining location (12a) is rotated relative to a second joining location (12b) about the common axis of rotation L.

2. Medical device according to claim 1,
**characterised in that**
the holding element (20) has a flexible guide means, especially a guide wire (21).

3. Medical device according to claim 1 or 2,
**characterised in that**
the lattice structure (10) has a first axial end (13a) and a second axial end (13b), the first axial end (13a) being joined to the holding element (20) for conjoint rotation therewith and the second axial end (13b) being freely arranged.

4. Medical device according to claim 3,
**characterised in that**
the first joining location (12a) is arranged at the second axial end (13b) and the second joining location (12b) is arranged at the first axial end (13a).

5. Medical device according to at least one of claims 1 to 4,
**characterised in that**
the first filament (11 a) is braided with the second filament (11 b).

6. Medical device according to at least one of claims 1 to 5,
**characterised in that**
the lattice structure (10) includes a plurality of first filaments (11 a) and/or a plurality of second filaments (11 b), wherein at least regionally the first filaments (11 a) have the same first twist angle α in each case and the second filaments (11b) have the same second twist angle β in each case.

7. Medical device according to at least one of claims 1 to 6,
**characterised in that**
the first twist angle α and/or the second twist angle β vary at least regionally along the lattice structure (10).

8. Medical device according to at least one of claims 1 to 7,
**characterised in that**
the lattice structure (10) has at least one first longitudinal region (14a) and at least one second longitudinal region (14b), the twist angles α, β in the first longitudinal region (14a) and in the second longitudinal region (14b) being set so that the first longitudinal region (14a) is, on changing of the lattice structure (10) from the radially compressed to the radially expanded state, or vice-versa, rotated about the common axis of rotation L in the opposite direction to the second longitudinal region (14b).

9. Medical device according to claim 8,
**characterised in that**
the first filament (11 a) and the second filament (11 b) have respectively different regional lengths in the first longitudinal region (14a) and in the second longitudinal region (14b) and have substantially the same overall length over the complete length of the lattice structure (10).

10. Medical device according to claim 8 or 9,
**characterised in that**
the first twist angle α is, in the first longitudinal region (14a), larger and, in the second longitudinal region (14b), smaller than the second twist angle β.

11. Medical device according to at least one of claims 1 to 10,
**characterised in that**
the filaments (11a, 11b) have, at least regionally, at least one cutting portion, especially a cutting surface or a cutting edge.

12. Medical device according to at least one of claims 1 to 11,
**characterised in that**
the lattice structure (10) has a funnel-shaped region (15) in the vicinity of the first axial end.

13. Medical device according to at least one of claims 1 to 12,
**characterised in that**
the lattice structure (10), especially the funnel-shaped region (15), has, at least regionally, a fluid-tight covering.

14. Medical device having a first lattice structure (10b) according to one of the preceding claims and having at least one second lattice structure (10a) according to one of the preceding claims which is coaxially arranged within the first lattice structure (10b), the filaments (11a, 11b) of the first lattice structure (10b and the filaments (11c, 11d) of the second lattice structure (10a having twist angles α, β, α', β' that differ in such a way that, on changing of the lattice structures (10a, 10b) from the radially compressed to the radially expanded state, or vice-versa, the first lattice structure (10b) and the second lattice structure (10a) are arranged to be rotated relative to one another.

15. Medical device according to claim 14,
**characterised in that**
the first lattice structure (10b) and the second lattice structure (10a) each have at least two filaments (11a, 11b, 11c, 11d) wound in the same direction around the common axis of rotation, the filaments (11a, 11b) of the first lattice structure (10b) being, at least at the common second axial end (13b) of the lattice structures (10a, 10b), wound in the opposite direction about the common axis of rotation relative to the filaments (11c, 11d) of the second lattice structure (10a), in order to form a grasping region (22) actuatable in the manner of scissors or pincers.

16. Method of producing a medical device according to claim 1, comprising the following steps:
- providing a braiding machine which has bobbins rotating about a common axis of rotation L,
- joining the first filament (11a) to a first bobbin and the second filament (11b) to a second bobbin,
- setting the rotation of the bobbins in such a way that the first filament (11a) is wound at the first twist angle α and the second filament (11 b) at the second twist angle β in opposite directions about the common axis of rotation L so as to form the rotationally symmetrical lattice structure (10),
- joining the first filament (11a) to the second filament (11b) at the first joining location (12a) and at the second joining location (12b), which is arranged spaced apart from the first joining location (12a)
or
turning back a common wire element (11) forming the first filament (11a) and the second filament (11b) so as to form the first joining location (12a) and joining a respective free end of the first filament (11 a) with a free end of the second filament (11b) so as to form the second joining location (12b), which is arranged spaced apart from the first joining location (12a);
- joining a first axial end (13a), especially a proximal end, of the lattice structure (10) to the holding element (20).

17. Medical device for detaching concretions in hollow organs of the body, having at least one rotationally symmetrical lattice structure (10) which is joined to a holding element (20) for conjoint rotation therewith and which has at least two lattice webs (11 a, 11 b) which cross one another and are helically arranged around a common axis of rotation L and which are joined to one another at at least two joining locations (12a, 12b) arranged spaced apart in the longitudinal direction of the lattice structure (10), a first lattice web (11a) forming a first web angle α and a second lattice web (11 b) forming a second web angle β which, at least regionally, differs from the first web angle α in such a way that, on changing of the lattice structure (10) from a radially compressed to a radially expanded state, or vice-versa, a first joining location (12a) is rotated relative to a second joining location (12b) about the common axis of rotation L.

18. Treatment system comprising a medical device according to one of the preceding claims 1 to 15 and 17 and a catheter in which the holding element (20) is arranged so as to be longitudinally displaceable, the holding element (20) being joined to a first axial end (13a) of the lattice structure (10), which end faces a proximal end of the catheter.

19. Method of producing a treatment system according to claim 18, comprising the following steps:
- providing a sheath having a proximal end and a distal end;
- arranging the holding element (20) in the sheath, the holding element (20) being guided from the distal end to the proximal end of the sheath;
- changing the lattice structure (10) from a radially expanded to a radially compressed state by drawing the lattice structure (10) into the distal end of the sheath, whereupon the second joining location (12b) of the lattice webs, especially of the filaments (11a, 11b), especially that arranged at a second, free axial end (13b) of the lattice structure (10), is rotated relative to the holding element (20) about the common axis of rotation L.

## Revendications

1. Dispositif médical pour dégager des calculs dans des organes corporel creux, comportant au moins une structure en treillis symétrique en rotation (10) qui est reliée sans pouvoir tourner à un élément de retenue (20) et au moins deux filaments (11a, 11b) enroulés autour d'un axe de rotation commun L et se croisant, qui sont reliés l'un à l'autre sur au moins deux sites de liaison (12a, 12b) disposés à distance dans la direction longitudinale de la structure en treillis (10), dans lequel un premier filament (11a) forme un premier angle de tresse α et un deuxième filament (11 b) forme un deuxième angle de tresse β, qui est différent au moins partiellement du premier angle de tresse α, de telle sorte qu'un premier site de liaison (12a) lors du passage de la structure en treillis (10) d'un état comprimé radialement à un état déployé radialement ou inversement, soit tourné par rapport à un deuxième site de liaison (12b) autour de l'axe de rotation commun L.

2. Dispositif médical selon la revendication 1,
**caractérisé en ce que**
l'élément de retenue (20) présente un moyen de guidage flexible, en particulier, un fil-guide (21).

3. Dispositif médical selon la revendication 1 ou 2,
**caractérisé en ce que**
la structure en treillis (10) présente une première extrémité axiale (13a) et une deuxième extrémité axiale (13b), dans lequel la première extrémité axiale (13a) est reliée sans pouvoir tourner à l'élément de retenue et la deuxième extrémité axiale (13b) est disposée librement.

4. Dispositif médical selon la revendication 3,
**caractérisé en ce que**
le premier site de liaison (12a) est disposé sur la deuxième extrémité axiale (13b) et le deuxième site de liaison (12b) est disposé sur la première extrémité axiale (13a).

5. Dispositif médical selon au moins l'une des revendications 1 à 4,
**caractérisé en ce que**
le premier filament (11a) est tressé avec le deuxième filament (11b).

6. Dispositif médical selon au moins l'une des revendications 1 à 5,
**caractérisé en ce que**
la structure en treillis (10) comprend plusieurs premiers filaments (11a) et/ou plusieurs deuxièmes filaments (11b), dans lequel au moins partiellement, les premiers filaments (11a) présentent respectivement le même premier angle de tresse α et les deuxièmes filaments (11b) présentent respectivement le même deuxième angle de tresse β.

7. Dispositif médical selon au moins l'une des revendications 1 à 6,
**caractérisé en ce que**
le premier angle de tresse α et/ou le deuxième angle de tresse β varie au moins partiellement le long de la structure en treillis (10).

8. Dispositif médical selon au moins l'une des revendications 1 à 7,
**caractérisé en ce que**
la structure en treillis (10) présente au moins un premier segment longitudinal (14a) et au moins un deuxième segment longitudinal (14b), dans lequel les angles de tresse α, β dans le premier segment longitudinal (14a) et dans le deuxième segment longitudinal (14b) sont ajustés de telle sorte que le premier segment longitudinal (14a), lors du passage de la structure en treillis (10) d'un état comprimé radialement à un état déployé radialement ou inversement, soit tourné en sens inverse par rapport au deuxième segment longitudinal (14b) autour de l'axe de rotation commun L.

9. Dispositif médical selon la revendication 8,
**caractérisé en ce que**
le premier filament (11a) et le deuxième filament (11 b) présentent dans le premier segment longitudinal (14a) et dans le deuxième segment longitudinal (14b) respectivement des longueurs de segments différentes et présentent sur la longueur totale de la structure en treillis (10) essentiellement la même longueur totale.

10. Dispositif médical selon la revendication 8 ou 9,
**caractérisé en ce que**
le premier angle de tresse α est plus grand dans le premier segment longitudinal (14a) et est plus petit dans le deuxième segment longitudinal (14b) que le deuxième angle de tresse β.

11. Dispositif médical selon au moins l'une des revendications 1 à 10,
**caractérisé en ce que** les filaments (11a, 11b) présentent au moins partiellement, au moins une zone de coupe, en particulier une surface de coupe ou un bord de coupe.

12. Dispositif médical selon au moins l'une des revendications 1 à 11,
**caractérisé en ce que**
la structure en treillis (10) présente dans la zone de la première extrémité axiale, un segment en forme d'entonnoir (15).

13. Dispositif médical selon au moins l'une des revendications 1 à 12,
**caractérisé en ce que**
la structure en treillis (10), en particulier, le segment en forme d'entonnoir (15) présente au moins partiellement un revêtement étanche aux fluides.

14. Dispositif médical comportant une première structure en treillis (10b) selon l'une des revendications précédentes et au moins une deuxième structure en treillis (10a) selon l'une des revendications précédentes, qui est disposée sur le plan coaxial à l'intérieur de la première structure en treillis (10b), dans lequel les filaments (11a, 11b) de la première structure en treillis (10b) présentent par rapport aux filaments (11 c, 11 d) de la deuxième structure en treillis (10a) des angles de tresse différents α, β, α', β' de telle sorte que lors du passage des structures en treillis (10a, 10b) de l'état comprimé radialement à l'état déployé radialement ou inversement, la première structure en treillis (10b) et la deuxième structure en treillis (10a) puissent tourner l'une par rapport à l'autre.

15. Dispositif médical selon la revendication 14,
**caractérisé en ce que**
la première structure en treillis (10b) et la deuxième structure en treillis (10a) présentent respectivement au moins deux filaments (11a, 11b, 11c, 11d) enroulés dans le même sens autour de l'axe de rotation commun, les filaments (11a, 11b) de la première structure en treillis (10b) étant enroulés au moins à la deuxième extrémité axiale commune (13b) des structures en treillis (10a, 10b) par rapport aux filaments (11 c, 11 d) de la deuxième structure en treillis (10a) en sens contraires autour de l'axe de rotation commun, pour former un segment de préhension (22) pouvant être actionné comme des ciseaux ou comme une pince.

16. Procédé de production d'un dispositif médical selon la revendication 1, comprenant les étapes suivantes :
- mise à disposition d'une machine de tressage qui présente des fuseaux tournant autour d'un axe de rotation commun L ;
- liaison du premier filament (11a) avec un premier fuseau et du deuxième filament (11b) avec un deuxième fuseau ;
- réglage de la rotation des fuseaux de telle sorte que le premier filament (11a) avec le premier angle de tresse α et que le deuxième filament (11b) avec le deuxième angle de tresse β soient enroulés, pour former la structure en treillis symétrique en rotation (10), en sens inverse autour de l'axe de rotation commun L;
- liaison du premier filament (11a) avec le deuxième filament (11 b) au premier site de liaison (12a) et au deuxième site de liaison (12b) disposé à distance du premier site de liaison (12a)
ou
déviation d'un élément de fil commun (11) qui forme le premier filament (11a) et le deuxième filament (11b) pour former le premier site de liaison (12a) et liaison respectivement d'une extrémité libre du premier filament (11a) avec une extrémité libre du deuxième filament (11 b) pour former le deuxième site de liaison (12b) disposé à distance du premier site de liaison (12a) ;
- liaison d'une première extrémité axiale (13a), en particulier d'une extrémité proximale, de la structure en treillis (10) avec l'élément de retenue (20).

17. Dispositif médical pour dégager des calculs dans des organes corporel creux, comportant au moins une structure en treillis symétrique en rotation (10) qui est reliée sans pouvoir tourner à un élément de retenue (20) et au moins deux traverses de treillis (11a, 11b) disposées en spirale autour d'un axe de rotation commun L et se croisant, qui sont reliées l'une à l'autre sur au moins deux sites de liaison (12a, 12b) disposés à distance dans la direction longitudinale de la structure en treillis (10), dans lequel une première traverse de treillis (11 a) forme un premier angle de traverse α et une deuxième traverse de treillis (11 b) forme un deuxième angle de traverse β, qui est différent au moins partiellement du premier angle de traverse α, de telle sorte qu'un premier site de liaison (12a) lors du passage de la structure en treillis (10) d'un état comprimé radialement à un état déployé radialement ou inversement, soit tourné par rapport à un deuxième site de liaison (12b) autour de l'axe de rotation commun L.

18. Système de traitement avec un dispositif médical selon l'une des revendications 1 à 15 et 17 précédentes, et un cathéter dans lequel l'élément de retenue (20) est disposé de façon à pouvoir coulisser longitudinalement, dans lequel l'élément de retenue (20) est relié à une première extrémité axiale (13a) de la structure en treillis (10), qui est orientée vers une extrémité proximale du cathéter.

19. Procédé de production d'un système de traitement selon la revendication 18, comprenant les étapes suivantes :
- mise à disposition d'un sas comportant une extrémité proximale et une extrémité distale ;
- disposition de l'élément de retenue (20) dans le sas, l'élément de retenue (20) étant conduit de l'extrémité distale à l'extrémité proximale du sas ;
- passage de la structure en treillis (10) d'un état déployé radialement à un état comprimé radialement par insertion de la structure en treillis (10) dans l'extrémité distale du sas, dans lequel le deuxième site de liaison (12b) disposé en particulier sur une deuxième extrémité axiale libre (13b) de la structure en treillis (10) des traverses de treillis, en particulier des filaments (11a, 11 b) est tourné par rapport à l'élément de retenue (20) autour de l'axe de rotation commun L.
